# EUROPEAN PATENT SPECIFICATION

(11) **EP 2 678 690 B1**
(45) Date of publication and mention of the grant of the patent: **24.01.2018**
(21) Application number: 12705303.1
(22) Date of filing: 22.02.2012
(51) Int. Cl.: G01N 33/68

(54) **METHOD AND USE OF METABOLITES FOR THE DIAGNOSIS OF INFLAMMATORY BRAIN INJURY IN PRETERM BORN INFANTS**
VERFAHREN UND VERWENDUNG VON METABOLITEN ZUR DIAGNOSE VON ENTZÜNDLICHER HIRNLÄSION BEI FRÜHGEBURTEN
UTILISATION DE MÉTABOLITES POUR LE DIAGNOSTIC DE LÉSIONS CÉRÉBRALES INFLAMMATOIRES CHEZ LES NOUVEAU-NÉS PRÉMATURÉS ET PROCÉDÉ ASSOCIÉ

(30) Priority: 22.02.2011 EP 11155450
(43) Date of publication of application: 01.01.2014
(73) Proprietor: InfanDx AG, 50670 Köln (DE)
(72) Inventor: KELLER, Matthias, 45219 Essen (DE); MALLARD, Carina, Box 432 S-405 30 Gothenburg (SE); DEIGNER, Hans-Peter, 68623 Lampertheim (DE); ENOT, David, F-14480 Creully (FR); IGWE, Emeka I., 80333 München (DE)
(74) Representative: Winter, Brandl, Fürniss, Hübner, Röss, Kaiser, Polte - Partnerschaft mbB
(86) International application number: PCT/EP2012/053016
(87) International publication number: WO 2012/113834

(56) References cited:
- US-A1- 2007 004 044
- MENKES J H ET AL: "Effect of elevated blood tyrosine on subsequent intellectual development of premature infants.", THE JOURNAL OF PEDIATRICS OCT 1966 LNKD- PUBMED:5921333, vol. 69, no. 4, October 1966 (1966-10), pages 583-588, XP009147929, ISSN: 0022-3476
- YURDAKOK MURAT ET AL: "Cerebrospinal fluid amino acid levels in newborn infants with intracranial hemorrhage", ACTA PAEDIATRICA JAPONICA, vol. 37, no. 6, 1995, pages 694-696, XP009147900, ISSN: 0374-5600
- MUELLER P ET AL: "Mass Spectrometric Quantifications Of Organic Acids And Acylcarnitines In Early Random Urine Specimens Of Newborns With Perinatal Complications: Feasibility Study For The Prediction Of The Neuro developmental Outcome", THE INTERNET JOURNAL OF PEDIATRICS AND NEONATOLOGY, INTERNET SCIENTIFIC PUBLICATIONS, LLC, US , vol. 7, no. 2 1 January 2007 (2007-01-01), pages 1-15, XP002546473, internet ISSN: 1528-8374 Retrieved from the Internet: URL:http://www.ispub.com/journal/the_inter net_journal_of_pediatrics_and_n eonatology/volume_7_number_2_9/article/mas s_spectrometric_quantificat ions_of_organic_acids_and_acylcarnitines_i n_early_random_urine_specim ens_of_newborns_with_perinatal_complicatio ns_feasibility_study_for_th e_prediction_of_the_ [retrieved on 2009-07-06] cited in the application
- KENNY LOUISE C ET AL: "Robust Early Pregnancy Prediction of Later Preeclampsia Using Metabolomic Biomarkers", HYPERTENSION, LIPPINCOTT WILLIAMS & WILKINS, US, vol. 56, no. 4, 1 October 2010 (2010-10-01), pages 741-749, XP009145728, ISSN: 0194-911X, DOI: DOI:10.1161/HYPERTENSIONAHA.110.157297
- Matthias Keller ET AL: "Inflammatory-Induced Hibernation in the Fetus: Priming of Fetal Sheep Metabolism Correlates with Developmental Brain Injury", PLoS ONE, vol. 6, no. 12, 29 December 2011 (2011-12-29), page e29503, XP055127566, ISSN: 1932-6203, DOI: 10.1371/journal.pone.0029503
- FIFE W P ET AL: "Susceptibility of fetal sheep to acute decompression sickness.", UNDERSEA BIOMEDICAL RESEARCH SEP 1978, vol. 5, no. 3, September 1978 (1978-09), pages 287-292, ISSN: 0093-5387
- NATH JAY ET AL: "Metabolomic perfusate analysis during kidney machine perfusion: the pig provides an appropriate model for human studies.", PLOS ONE 2014, vol. 9, no. 12, 2014, page e114818, ISSN: 1932-6203

## Description

The present invention relates to a method for predicting the likelihood of an onset of an inflammation-patient *in vitro* in accordance with claim 1, and a use of a plurality of endogenous metabolites from a blood sample *in vitro* for predicting the likelihood of an onset of an infection leading to brain injury in preterm born infants according to claim 11.

The invention generally relates to biomarkers for brain injury in preterm born infants as tools in clinical diagnosis for early detection of brain injury in preterm born infants, brain injury in preterm born infants therapy monitoring and methods based on the same biomarkers.

### BACKGROUND of the Invention

Brain injury in preterm infants, a major problem of prematurity is one of the major causes of lifelong neurological sequelae, with a lack of early diagnostic tools. Identification of early biomarkers for outcome could enable physicians and scientists to develop targeted pharmacological and behavioural therapies to restore functional connectivity

The societal suffering from perinatal brain damage is immense. Roughly 1-2 out of 200 births occur very prematurely (<30 weeks gestation, birth weight <1000g). The disability rate in this gestational age group is very high; among extremely low gestational age newborns (ELGANs, <28 weeks) the rate may be as high as 80%. In essence, the prevalence at birth of disability associated with extreme prematurity might be considerably higher than 1 in 1.000.

In the EU, approximately 60.000 infants are born each year that sustain some sort of brain injury. Improved capabilities to protect or even intervene in this scenario are much desired. Especially the high risk group of preterm infants ranges from 4-6% to up to 10% of all newborns in developed Western countries. Annually, approximately 4 million live births occur in the EU (source: EUROSTAT). Thus, approximately 200.000 infants will be preterm, and about 15-20.000 will be extremely preterm (<28 weeks gestation). In the United States, approximately 4 million live births occur annually. Among these are 499.000 (12.3%) preterm infants, 30.000 being <28weeks gestation.

In the past decades there was a continuous rise in the rate of preterm deliveries [Greene MF. Outcomes of very low birth weight in young adults. NEJM 2002; 346:146-148]. In Tyrol the annual number of live births is approximately 6.800, 10% of newborn babies (n=670) are preterm infants and more than 1% (n=80) are extremely preterm infants with a gestational age of less than 32 weeks. Advances in perinatal medicine resulted in a dramatic increase in survival of these infants [Hack M, Taylor HG, Drotar D, et al. Chronic conditions, functional limitations, and special health care needs of school-aged children born with extremely low-birth-weight in the 1990s. JAMA 2005; 294:318-325; Anderson P, Doyle LW. Victorian Infant Collaborative Study Group. Neurobehavioral outcomes of school-age children born extremely low birth weight or very preterm in the 1990s. JAMA 2003; 289:3264-3272; Hack M, Klein N. Young adult attainments of preterm infants. JAMA 2006; 295:695-696]. But there is still a substantial rate of morbidity among surviving very preterm infants, especially concerning brain damage resulting in neurodevelopmental impairments [Farooqi A, Hagglof B, Sedin G, et al. Chronic conditions, functional limitations, and special health care needs in 10- to 12-year-old children born at 23 to 25 weeks' gestation in the 1990s: a Swedish national prospective follow-up study. Pediatrics 2006; 118:e1466-e1477; Mikkola K, Ritari N, Tommiska V, et al. Neurodevelopmental outcome at 5 years of age of a national cohort of extremely low birth weight infants who were born in 1996-1997. Pediatrics 2005; 116:1391-1400]. 20-40% of these infants suffer from chronic conditions requiring special services and therapies [7. Resnick MB, Gomatam SV, Carter RL, et al. Educational disabilities of neonatal intensive care graduates. Pediatrics 1998; 1002:308-314].

The most important source of societal suffering from perinatal brain damage is on the individual and family level. Four out of five preterm infants are limited in their everyday activities. Moreover, brain-damage-associated cognitive and learning difficulties represent a potentially preventable source of suffering.

Prevention of perinatal brain damage, therefore, is of major importance for public health and obviously for individual well being. Both white and grey matter are affected in perinatal brain damage in preterm infants. Long term-consequences of extreme prematurity and the associated brain damage are devastating, including cognitive, motor, perception and learning limitations. The current pathogenetic paradigm of perinatal brain damage in preterm infants has multiple inter-related aspects and includes exposure to infection/ inflammation, hypoxia-ischemia, and free radicals. It is likely that these mechanisms act in concert.

Inflammation and perturbations to cytokine signalling are associated with adverse pregnancy outcomes, such as miscarriage, pre-eclampsia, preterm labour and foetal brain injury.

Intrauterine inflammation in particular has been associated with adverse neurologic outcomes in preterm infants, the precise mechanisms of fetal brain injury remain unclear. The presence of increased macrophages and increased circulating interleukin 6 levels in LPS-exposed pups suggests that these are significant factors associated with potential brain damage associated with intrauterine inflammation and preterm birth. [Ernst LM, Gonzalez J, Ofori E, Elovitz M. Inflammation-induced preterm birth in a murine model is associated with increases in fetal macrophages and circulating erythroid precursors. Pediatr Dev Pathol. 2010, 13: 273-81].

Recent experimental studies that have examined the effects of lipopolysaccharide (LPS) exposure to the fetus or neonate (such as the consequences of intracerebral LPS injections) and the interaction of LPS with other events show the induction of a marked cerebral cytokine response and prominent white matter lesions. LPS administered intravenously to the fetus also induces gross lesions, which are mainly localised to the white matter and are accompanied by activation of inflammatory cells. Cerebral effects following fetal LPS exposure via more distant routes, such as intracervical, intrauterine or maternal LPS administration, are characterised by reductions in oligodendrocyte or myelin markers without macroscopic lesions being evident.

Further, pro-inflammation at birth is associated with changes in the IGF-system, one factor suspected to be relevant to the development of brain damage in preterm infants. Hansen-Pupp I, Hellström-Westas L, Cilio CM, Andersson S, Fellman V, Ley D. Inflammation at birth and the insulin-like growth factor system in very preterm infants Acta Paediatr. 2007, 96: 830-6.

Additional evidence that inflammation and lipopolysaccharide-induced preterm births is responsible for irreversible neuronal injury is provided by reports such as e.g. by a paper published by Burd et al. [Burd I, Chai J, Gonzalez J, Ofori E, Monnerie H, Le Roux PD, Elovitz MA. Beyond white matter damage: fetal neuronal injury in a mouse model of preterm birth. Am J Obstet Gynecol. 2009, 201: 279.e1-8].

Accordingly an early detection of preterm inflammation-related brain injury will enable a more effective therapeutic treatment with a correspondingly more favourable clinical outcome. To meet the above needs, the prior art provides some approaches to early prognostic evaluations of brain injury in preterm born infants, none of which however, is specific to preterm infants and inflammation and has lead to any clinical relevance, let alone to true reliability.

Early detection of a disease based on symptoms as currently common in clinics is problematic since it is not reliable and the disease may have progressed before diagnosis is possible. Preterm brain injury is one such class of diseases and still is assessed with diagnostic approaches such as the Apgar score to summarily assess the health of newborns after birth. The Apgar score (ranging from zero to 10) is determined by evaluating the newborn baby on five simple criteria then summing up the five values thus obtained. [cf. Apgar, Virginia, A proposal for a new method of evaluation of the newborn infant, Curr. Res. Anesth. Analg. 1953, 32: 260-267. Finster M; Wood M., The Apgar score has survived the test of time". Anesthesiology 2005, 102: 855-857].

However, the reliable diagnosis of preterm brain damage still remains a challenge. Potential marker of periventricular white matter injury in preterm currently include proteins such as increased maternal/fetal blood S100B levels, a protein, however which changes along with a great variety of states of diseases. [Garnier Y, Frigiola A, Li Volti G, Florio P, Frulio R, Berger R, Alm S, von Duering MU, Coumans AB, Reis FM, Petraglia F, Hasaart TH, Abella R, Mufeed H, Gazzolo D. Increased maternal/fetal blood S100B levels following systemic endotoxin administration and periventricular white matter injury in preterm fetal sheep Reprod Sci. 2009, 16: 758-66.]

Other options to gain insights into brain injury and outcomes in premature infants are expensive and technologically demanding imaging techniques such as Magnetic resonance imaging (MRI) [Mathur A, Inder T. MRI include diffusion tensor imaging and sophisticated image analysis tools, such as functional connectivity, voxel-based morphometry, J Commun Disord. 2009, 42: 248-55].

Currently used diagnostic methods thus require time and appropriate equipment with high costs and frequently unsatisfying sensitivities. However, these used diagnostic means have major limitations such as reduced area under the curve (AUC) and/or delay of diagnosis or increased costs due to equipment required. Accordingly these procedures do not allow a timely assessment of an acute and rapidly evolving disease and overall the situation is far from satisfying and from providing a rapid and reliable diagnosis of preterm brain damage; there is still an urgent need for differentiation of brain injury from any other state of health as a prerequisite for timely treatment.

In classical patient screening and diagnosis, the medical practitioner uses a number of diagnostic tools for diagnosing a patient suffering from a certain disease. Among these tools, measurement of a series of single routine parameters, e.g. in a blood sample, is a common diagnostic laboratory approach. These single parameters comprise for example enzyme activities and enzyme concentration and/or enzyme detection

As far as such diseases are concerned which easily and unambiguously can be correlated with one single parameter or a few number of parameters achieved by clinical chemistry, these parameters have proved to be indispensable tools in modern laboratory medicine and diagnosis. However, in complex pathophysiological conditions, which share a lack of an unambiguously assignable single parameter or marker, differential diagnosis from blood or tissue samples is currently difficult to impossible.

WO 2011/012553 A1 claiming priority from EP 09167018.2 filed on 31. July 2009 by the present applicant, having the title "Method for predicting the likelihood of an onset of an inflammation associated organ failure". In particular, WO 2011/012553 A1 analyzes in a biological sample of a mammalian subject in vitro by means of quantitative metabolomics analysis. In particular, WO 2011/012553 A1 determines the concentration of acylcarnitines, sphingomyelins, hexoses and glycerophospholipids in brain homogenates and in plasma by means of (FIA-MS/MS). Furthermore, amino acids and biogenic amines were analyzed by reversed phase LC-MS/MS in brain homogenates and in plasma.

Additionally, WO 2011/012553 A1 determines prostanoids - a term summarizing prostaglandins (PG), thromboxanes (TX) and prostacylines - and oxidised fatty acid metabolites in plasma extracts by LC-ESI-MS/MS and in brain homogenate extracts by online solid phase extraction (SPE)-LC-MS/MS.

Furthermore, energy metabolism (Organic Acids) (LC-MS/MS) was analyzed in WO 2011/012553 A1: For the quantitative analysis of energy metabolism intermediates (glycolysis, citrate cycle, pentose phosphate pathway, urea cycle) hdyrophilic interaction liquid chromatography (HILIC)-ESI-MS/MS method (plasma and brain homogenates).

Experiments in WO 2011/012553 A1 were carried out with samples from human beings suffering from sepsis (systemic infection) of known (peritonitis and pneumonia) and unknown origin developing a sepsis related organ failure, and in animal testing, a sepsis mouse model and an induced liver failure model in mice were subject of testing.

Furthermore, WO 2010/128054 A1 and EP 2 249 161 A1, filed on 5. May 2009, by the present applicant disclose a method of diagnosing asphyxia.

In particular, WO 2010/128054 A1 relates to a method for in vitro diagnosing e.g. perinatal asphyxia and disorders related to hypoxia,
characterized by quantitatively detecting in at least one biological sample of at least one tissue of a mammalian subject a plurality of asphyxia specific compounds having a molecular weight of less than 1500 Dalton, except lactate, comprising the steps of:
a) selecting said compounds;
b) measuring at least one of the parameters selected from the group consisting of: concentration, level or amount of each individual metabolite of said plurality of metabolites in said sample, qualitative and/or quantitative molecular pattern and/or molecular signature; and using and storing the obtained set of values in a database;
c) calibrating said values by comparing asphyxia-positive and/or asphyxia-negative reference parameters;
d) comparing said measured values in the sample with the calibrated values, in order to assess whether the patient is asphyxia-positive or asphyxia-negative.

The method according to WO 2010/128054 A1 uses asphyxia specific compounds as biomarkers which are endogenous compounds being selected from the group consisting of: biogenic amines; carnitine-derived compounds; amino acids; bile acids; carboxylic acids; eicosanoids; lipids; precursors of cholesterol, cholesterol metabolites, prostanoids; and sugars.

Furthermore, WO 2010/128054 A1 relates to a method of in vitro estimating duration of hypoxia in a patient, a method for in vitro monitoring of normoxic, hypoxic and hyperoxic conditions and/or normobaric and hyperbaric oxygen therapy and a kit for carrying out the methods thereof.

However, neither the group of preterm born neonates, nor brain damage other than hypoxic-ischemic encephalopathy in neonates is addressed with the studies carried out in WO 2011/012553 A1 and WO 2010/128054 A1.

Finally, Solberg R, Enot, D, Deigner, H-P, Koal, T, Scholl-Bürgi, S, Saugstad, OD, and Keller, M (2010): "Metaboloic Analyses of Plasma Reveals New Insights into Asphyxia and Resuscitation in Pigs", PLoS ONE 5(3): e9606. doi:10.1371/journal.pone.0009606 disclose detection of a number of metabolites in plasma taken before and after hypoxia as well as after resuscitation, in asphyxiated mice, in order to evaluate pathophysiological mechanisms of hypoxemia in newborns. Hypoxemia of different durations was induced in newborn piglets before randomization for resuscitation with 21% or 100% oxygen for 15 min or polonged hyperoxia in order to indicate the duration and/or severity of hypoxia and finally finding a risk assessment for hypoxic-ischemic encephalopathy (HIE) in patients suffering from perinatal asphyxia. The metabolites of the study of Solberg et al. [2010] includes amino acids, particularly branched chained amino acids, metabolites of the Krebs cycle, including α-ketoglutarate, succinate and fumarate, biogenic amines, bile acids, prostaglandins, sphingolipids, glycerophospholipids, oxysterols and acylcarnitines.

Assessment of brain injuries per se, or biomarkers to detect brain injuries per se are not comprised by the Solberg et al. [2010] paper (cf. Solberg [2010] et al., page 9, left column, lines 2-3).

Additionally, Mueller P, Robel-Tilling, E, Hueckel, D, Ceglarek and Vogtmann, C (2007): "Mass Spectrometric Quantifications of Organic Acids and Acylcarnitines in Early Random Urine Specimens of Newborns with Perinatal Complications: Feasability Study for the Prediction of the Neuro-Developmental Outcome", The Internet Journal of Pediatrics and Neonatology, Vol. 7, No. 2 describe the use of MS quantifications of Organic Acids and Acylcarnitines for the Prediction of the Neuro-Developmental Outcome in newborns with perinatal complications.

In particular, Mueller et al. [2007] investigated in a group of preterm infants a number of 65 quantitatively detected metabolites (42 organic acids, 22 acylcarnitines, free carnitine and 15 ratios) in urine within the first 72 hours of life of preterms. Reliable prediction for development of HIE (hypoxic-ischemic encephalopathy) caused by severe asphyxia was demonstrated with metabolite monitoring of the lactic acid/creatinine ratio in urine of asphyxiated newborns. However, an unexpected result of the Mueller et al. [2007] study was the finding that the total amount of urinary acylcarnitines differed not significantly between the comparison group and the patient group with severe neurological defects.

A paper published by Menkes J H et al. relates to an analysis concerning the relationship between blood tyrosine concentration in preterm infant and intellectual development [J. Pediatr. 1966, vol. 69, no.4, 583-588].

A paper published by Yurdakok Murat et al. refers to an analysis of cerebrospinal amino fluid acid levels in connection with intracranial hemorrhage [Acta Paediatr. Jpn. 1995, vol. 37, no.6, 694-696].

A paper published by Mueller et al. is directed to an analysis of urine specimens in connection with neurodevelopment of newborns, such as social behaviour and hearing and visual abilities [Internet J Pediatr Neonatol. 2007, vol. 7, no.2, 1-15].

A paper of by Keller et al. published on December 29, 2011 relates to a study regarding the correlation of specific metabolites in blood to inflammatory-induced developmental brain injury (PLOS ONE 2011, vol. 6, issue 12, e29503).

To summarize, the quantitation of plasma metabolites by targeted quantitative metabolomics offers a new tool for biomarker discovery.

However, so far no metabolic markers have been introduced for indicating and diagnosing inflammation-related brain injury in preterms. Solely a couple of intermediates, possibly involved in pathobiochemistry, have been discussed in the wider context of brain damage and chemical mediators that may contribute to white matter injury include reactive oxygen and nitrogen species, glutamate, cytokines, and adenosine [Back SA, Rivkees SA Emerging concepts in periventricular white matter injury. Semin Perinatol. 2004, 28: 405-14].

Thus, it is the problem underlying the present invention to provide a diagnostic approach for inflammation-related brain injury in preterms. Additionally, the sample size of usually several ml of blood required for common diagnostic approaches is another problem of the invention, in particular for children and neonates and for continuous monitoring of critically ill subjects in general.

Given the remarkable and rapid potential of brain injury in preterm born infants to progress into an irreversible and life-threatening condition the current situation is highly problematic and unsatisfying and an early and reliable multiparameter diagnosis based on small sample sizes imperative.

The above mentioned problems are solved by a method in accordance with claim 1 and a use according to claim 11.

The current invention presents a solution to this problem ideally requiring only minute amounts of blood sample.

In particular, the present invention relates to a method for predicting the likelihood of inflammation-related brain injury in preterm born infants, with a greater than 70% accuracy,
characterized by
quantitatively detecting in vitro in at least one blood sample of a patient the following five compounds of a selection at time point 216h: Phenylthiocarbamyltyrosine, Lysophosphatidylcholine with acyl residue C28:1, wherein the number following "C" in the Lysophosphatidylcholine represents the number of carbon atoms in the residue, and the number after the colon represents the number of double bonds in the residue, Phenylalanine, Methionine sulfoxide and Putrescine, and being specific for inflammation-related brain injury, and having a molecular weight of less than 1500 Dalton comprising the steps of:
a) selecting further compounds from an endogenous metabolite group consisting of: Acetylcarnitine; Dodecanedioylcarnitine; Propionylcarnitine; Propenoylcarnitine; Butyrylcarnitine/Isobutyrylcarnitine; Butenoylcarnitine; Isovalerylcarnitine / 2-Methylbutyrylcarnitine / Valerylcarnitine; Glutaconylcarnitine / Mesaconylcarnitine; Glutarylcarnitine; Tetradecenoylcarnitine; 3-Hydroxyhexadecanoyl carnitine; Triglylcarnitine/ 3-Methyl-crotonylcarnitine; Phosphatidylcholine with diacyl residue sum C24:0; Phosphatidylcholine with diacyl residue sum C28:1; Phosphatidylcholine with diacyl residue sum C30:0; Phosphatidylcholine with diacyl residue sum C32:2; Phosphatidylcholine with diacyl residue sum C34:2; Phosphatidylcholine with diacyl residue sum C34:3; Phosphatidylcholine with diacyl residue sum C34:4; Phosphatidylcholine with diacyl residue sum C36:0; Phosphatidylcholine with diacyl residue sum C36:1; Phosphatidylcholine with diacyl residue sum C36:2; Phosphatidylcholine with diacyl residue sum C38:3; Phosphatidylcholine with diacyl residue sum C38:6; Phosphatidylcholine with diacyl residue sum C40:2; Phosphatidylcholine with diacyl residue sum C40:5; Phosphatidylcholine with diacyl residue sum C40:6; Phosphatidylcholine with acyl-alkyl residue sum C30:1; Phosphatidylcholine with acyl-alkyl residue sum C36:1; Phosphatidylcholine with acyl-alkyl residue sum C36:2; Phosphatidylcholine with acyl-alkyl residue sum C38:1; Phosphatidylcholine with acyl-alkyl residue sum C38:2; Phosphatidylcholine with acyl-alkyl residue sum C40:2; Tryptophane; Kynurenine; asymmetric dimethylarginine; symmetric dimethylarginine; total dimethylarginine; Phenylthiocarbamyl-methionine; Phenylthiocarbamyl-phenylalanine; Phenylthiocarbamyl-serine; Phenylthiocarbamyl-glycine; Glycine; Serine; Proline; Valine; Tyrosine, Citrulline;
b) measuring at least one of the parameters selected from the group consisting of: concentration, level or amount of each specific compound of said plurality of compounds in said sample, qualitative and/or quantitative molecular pattern and/or molecular signature; and storing the obtained set of values in a database;
c) calibrating said values by comparing clinically confirmed inflammation-related brain injury in preterm born infants-positive and/or clinically confirmed inflammation-related brain injury in preterm born infants-negative reference parameters; and
d) comparing said measured values in the sample with the calibrated values, in order to assess whether the preterm neonate patient is likely to develop an inflammation-related brain injury or is unlikely to develop an inflammation-related brain injury.

Furthermore, the present invention is directed to a use of the following five compounds of a selection at time point 216h: Phenylthiocarbamyltyrosine, Lysophosphatidylcholine with acyl residue C28:1, Phenylalanine, Methionine sulfoxide and Putrescine, for predicting the likelihood of an onset of an inflammation-associated brain injury in preterm born infants with a greater than 70% accuracy, from a blood sample in vitro, wherein the metabolites are selected from the group consisting of : Carnitine (free); Acetylcarnitine; Dodecanedioylcarnitine; Propionylcarnitine; Propenoylcarnitine; Butyrylcarnitine/Isobutyrylcarnitine; Butenoylcarnitine; Isovalerylcarnitine / 2-Methylbutyrylcarnitine / Valerylcarnitine; Glutaconylcarnitine / Mesaconylcarnitine; Glutarylcarnitine; Tetradecenoylcarnitine; 3-Hydroxyhexadecanoyl carnitine; Triglylcarnitine/ 3-Methyl-crotonylcarnitine; Phosphatidylcholine with diacyl residue sum C24:0; Phosphatidylcholine with diacyl residue sum C28:1; Phosphatidylcholine with diacyl residue sum C30:0; Phosphatidylcholine with diacyl residue sum C32:2; Phosphatidylcholine with diacyl residue sum C34:2; Phosphatidylcholine with diacyl residue sum C34:3; Phosphatidylcholine with diacyl residue sum C34:4; Phosphatidylcholine with diacyl residue sum C36:0; Phosphatidylcholine with diacyl residue sum C36:1; Phosphatidylcholine with diacyl residue sum C36:2; Phosphatidylcholine with diacyl residue sum C38:3; Phosphatidylcholine with diacyl residue sum C38:6; Phosphatidylcholine with diacyl residue sum C40:2; Phosphatidylcholine with diacyl residue sum C40:5; Phosphatidylcholine with diacyl residue sum C40:6; Phosphatidylcholine with acyl-alkyl residue sum C30:1; Phosphatidylcholine with acyl-alkyl residue sum C36:1; Phosphatidylcholine with acyl-alkyl residue sum C36:2; Phosphatidylcholine with acyl-alkyl residue sum C38:1; Phosphatidylcholine with acyl-alkyl residue sum C38:2; Phosphatidylcholine with acyl-alkyl residue sum C40:2; Tryptophane; Kynurenine; asymmetric dimethylarginine; symmetric dimethylarginine; total dimethylarginine; Phenylthiocarbamyl-methionine; Phenylthiocarbamyl-phenylalanine; Phenylthiocarbamyl-serine; Phenylthiocarbamyl-glycine; Glycine; Serine; Proline; Valine; Tyrosine, Citrulline.

In particular, the present invention provides a solution to these problems based on the application of a new technology in this context and on an unknown list of endogenous metabolites as diagnostic marker. Since metabolite concentration differences in blood samples provide links to the various phenotypical responses, metabolites are suitable biomarker candidates.

The present invention allows for accurate, rapid, and sensitive prediction and diagnosis of brain injury in preterm born infants through a measurement of a plurality of endogenous metabolic biomarker (metabolites) taken from a blood sample at a single point in time. This is accomplished by obtaining a biomarker panel at a single point in time from an individual, particularly an individual at risk of developing brain injury in preterm born infants, having brain injury in preterm born infants, or suspected of having brain injury in preterm born infants, and comparing the biomarker profile from the individual to reference biomarker values or scores. The reference biomarker values may be obtained from a population of individuals (a "reference population") who are, for example, afflicted with brain injury in preterm born infants or who are suffering from either the onset of inflammatory brain injury in preterm born infants or a particular stage in the progression of inflammatory brain injury in preterm born infants. If the biomarker panel values or score from the individual contains appropriately characteristic features of the biomarker values or scores from the reference population, then the individual is diagnosed as having a more likely chance of getting inflammatory brain injury in preterm born infants, as being afflicted with brain injury in preterm born infants or as being at the particular stage in the progression of brain injury in preterm born infants as the reference population.

Accordingly, the present invention provides, inter alia, methods of predicting the likelihood of an onset of inflammatory brain injury in preterm born infants in an individual. The methods comprises obtaining a biomarker score at a single point in time from the individual and comparing the individual's biomarker profile to a reference biomarker profile. Comparison of the biomarker profiles can predict the onset of brain injury in preterm born infants in the individual with an accuracy of at least about 70%. This method may be repeated again at any time prior to the onset of brain injury in preterm born infants.

The present invention further provides a method of determining the progression (i.e., the stage) of inflammatory brain injury in preterm born infants in an individual. This method comprises obtaining a biomarker profile composed of metabolite concentrations of the following five compounds of a selection at time point 216h: Phenylthiocarbamyltyrosine, Lysophosphatidylcholine with acyl residue C28:1, Phenylalanine, Methionine sulfoxide and Putrescine from the individual and comparing the individual's biomarker profile to a reference biomarker score. Comparison of the biomarker scores can determine the progression of inflammatory brain injury in preterm born infants in the individual with an accuracy of at least about 70%. This method may also be repeated on the individual at any time.

Additionally, the present invention provides a method of diagnosing inflammation-related brain injury in preterm born infants in an individual having or suspected of having brain injury in preterm born infants. This method comprises obtaining a biomarker score at a single point in time from the individual and comparing the individual's biomarker score to a reference biomarker score. Comparison of the biomarker profiles can diagnose brain injury in preterm born infants in the individual with an accuracy of at least about 70 %. This method may also be repeated on the individual at any time.

The present invention further provides, inter alia, a method of determining the status of inflammation-related brain injury in preterm born infants or diagnosing inflammatory brain injury in preterm born infants in an individual comprising obtaining a biomarker score from a blood sample taken from the individual and comparing the individual's metabolite biomarker score to a reference bile acid biomarker score. A single such comparison is capable of classifying the individual as having membership in the reference population. Comparison of the biomarker scores determines the status of brain injury in preterm born infants or diagnoses of inflammatory brain injury in preterm born infants in the individual.

In yet another embodiment, the present invention provides, inter alia, a method of determining the status of brain injury in preterm born infants or diagnosing brain injury in preterm born infants in an individual. The method comprises comparing a measurable characteristic of more than one biomarker between a metabolite biomarker panel or biomarker score composed by (processed or unprocessed) values of this panel obtained from a blood sample from the individual and a biomarker score obtained from blood samples from a reference population. Based on this comparison, the individual is classified as belonging to or not belonging to the reference population. The comparison, therefore, determines the likelihood of inflammatory brain injury in preterm born infants or diagnoses of such brain injury in preterm born infants in the individual.

The present invention provides methods for predicting inflammatory brain injury in preterm born infants. Such methods comprise the steps of: analyzing a blood sample from a subject to determine the levels of more than one biomarkers for inflammation-related brain injury in preterm born infants in the sample, where the biomarkers are following five compounds of a selection at time point 216h: Phenylthiocarbamyltyrosine, Lysophosphatidylcholine with acyl residue C28:1, Phenylalanine, Methionine sulfoxide and Putrescine and comparing the levels of the biomarkers, as well as a composed value / score generated by subjecting the concentrations of individual biomarkers in the sample to a classification method such as affording an equation to process single concentration values - to obtain a separation between both (diseased and healthy) groups or comparing the level(s) of the one or more biomarkers in the sample to brain injury in preterm born infants positive or brain injury in preterm born infants negative reference levels of the one or more biomarkers in order to determine whether the preterm subject is developing brain injury.

The present invention provides a solution to the problem described above, and generally relates to the use of metabolomics data, generated by quantitation of endogenous metabolites by but not limited to mass spectrometry (MS), in particular MS-technologies such as MALDI, ESI, atmospheric pressure chemical ionization (APCI), and other methods, determination of metabolite concentrations by use of MS-technologies or alternative methods coupled to separation (LC-MS, GC-MS, CE-MS), subsequent feature selection and combination of features to classifiers including molecular data of at least two molecules.

The concentrations of the individual markers, analytes, metabolites thus are measured and compared to reference values or data combined and processed to scores, classifiers and compared to reference values thus indicating diseased states etc. with superior sensitivities and specificities compared to known procedures, clinical parameters and biomarkers.

Those skilled in the art will understand that for the quantitation of certain metabolites, also chemically modified metabolites may be used as one may get a better separation on the column material used prior to the MS-technologies.

Furthermore, in some embodiments, the present invention provides a method of diagnosing brain injury in preterm born infants and/or duration/severity comprising: detecting (the presence or absence of 5 or more, 10 or more, etc. metabolites measured together in a multiplex or panel format) brain injury in preterm born infants specific metabolites in a blood sample from a subject; and diagnosing brain inflammatory injury in preterm born infants based on the presence of specific metabolites.

In a preferred embodiment of the invention, the method is characterized in that a deproteinization step and/or a separation step is inserted between steps a) and b), wherein said separation step is selected from the group consisting of liquid chromatography (LC), high performance liquid chromatography (HPLC), gas chromatography, liquid-liquid-extraction (LLE).

Said deproteinization step preferably is carried out by mixing said blood sample with organic solvents such as ethanol, methanol or acetonitrile.

In order to enhance sensitivity and/or volatility, e.g. for a better evaporation as used in mass spectrometry, the compounds can be derivatized as esters, amines or amides, wherein said derivatization includes: 2-Hydrazinopyridine (HP), 2-picolylamine (PA); Girard derivatization; oximation with hydroxylamine first and then silylation with hexamethyldisilazane and trifluoroacetic acid.

It is further preferred that said calibration step is carried out by
a) mathematically preprocessing said values in order to reduce technical errors being inherent to the measuring procedures used in accordance with the present invention, such as mass spectrometry.
b) selecting at least one suitable classifying algorithm from the group consisting of logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), inductive logic programming (ILP), generalized additive models, gaussian processes, regularized least square regression, self organizing maps (SOM), recursive partitioning and regression trees, K-nearest neighbor classifiers (K-NN), and applying said selected classifier algorithm to said preprocessed data of step a);
c) said classifier algorithms of step b) being trained on at least one training data set containing preprocessed data from subjects being divided into classes according to their inflammatory brain injury in preterm born infants-related pathophysiological, physiological, prognostic, or responder conditions , in order to select a classifier function to map said preprocessed data to said conditions;
d) applying said trained classifier algorithms of step c) to a preprocessed data set of a subject with unknown inflammatory brain injury in preterm born infants-related pathophysiological, physiological, prognostic, or responder condition, and using the trained classifier algorithms to predict the class label of said data set in order to predict the likelihood of an onset of inflammatory brain injury in preterm born infants of the subject.

The step of mathematically preprocessing can be carried out e.g. by means of a statistical method on obtained raw data, particularly raw intensity data obtained by a measuring device, wherein said statistical method is selected from the group consisting of raw data obtained by mass spectrometry or mass spectrometry coupled to liquid or gas chromatography or capillary electrophoresis or by 2D gel electrophoresis, quantitative determination with RIA or determination of concentrations/amounts by quantitation of immunoblots; smoothing, baseline correction, peak picking, optionally, additional further data transformation such as taking the logarithm in order to carry out a stabilization of the variances.

Furthermore, for reasons of better accuracy of the prognostic results, a further step of feature selection is inserted into said preprocessing step, in order to find a lower dimensional subset of features with the highest discriminatory power between classes; and/or
said feature selection is carried out by a filter and/or a wrapper approach;
and/or
wherein
said filter approach includes rankers and/or feature subset evaluation methods; and/or wherein said wrapper approach is applied, where a classifier is used to evaluate attribute subsets.

For the purpose of the present application, said pathophysiological condition corresponds to the label "diseased" and said physiological condition corresponds to the label "healthy" or said pathophysiological condition corresponds to different labels of "grades of a disease", "subtypes of a disease", different values of a "score for a defined disease"; said prognostic condition corresponds to a label "good", "medium", "poor", or "therapeutically responding" or "therapeutically non-responding" or "therapeutically poor responding".

Typically, the method of the present invention is characterized in that said measuring step is carried out by high-throughput mass spectrometry.
It is preferred, that said brain injury in preterm born infants specific endogenous compounds are inflammatory brain injury in preterm born infants specific endogenous metabolites.
Furthermore, in the method according to the present invention, typically, said mammalian subject is a human being, wherein raw data of metabolite concentrations are preprocessed using the log transformation;
wherein linear models are used to identify metabolites which are differentially present; wherein random forest (RF), K nearest neighbours (KNN), or linear discriminant analysis (LDA) is selected as suitable classifying algorithm, and is trained with preprocessed metabolite concentrations,
applying the obtained trained classifier to said preprocessed metabolite concentration data set of a subject under suspicion of having brain injury in preterm born infants, and using said trained classifier to predict or diagnose brain injury in preterm born infants A further embodiment of the present invention is a use of the following five compounds of a selection at time point 216h: Phenylthiocarbamyltyrosine, Lysophosphatidylcholine with acyl residue C28:1, Phenylalanine, Methionine sulfoxide and Putrescine
for carrying out a method for predicting the likelihood of an onset of inflammatory brain injury in preterm born infants and/or disorders related thereto in a mammalian subject.
a) detection agents for the detection of brain injury in preterm born infants specific endogenous metabolites, wherein said metabolites are the following five compounds of a selection at time point 216h: Phenylthiocarbamyltyrosine, Lysophosphatidylcholine with acyl residue C28:1, Phenylalanine, Methionine sulfoxide and Putrescine and
b) positive and/or negative controls; and
c) classification software for classification of the results achieved with said detection agents.

According to the present invention, the term "inflammation associated brain injury in preterm born infants" comprises " inflammatory brain damage and brain injury due to agents like toxins, cellular components of bacteria, fungi, viruses and parasites such as DNA or RNA fragments.
A preferred method is one, wherein the blood sample is plasma or serum.

Data classification is the categorization of data for its most effective and efficient use. Classifiers are typically deterministic functions that map a multi-dimensional vector of biological measurements to a binary (or n-ary) outcome variable that encodes the absence or existence of a clinically-relevant class, phenotype, distinct physiological state or distinct state of disease. To achieve this various classification methods such as, but not limited to, logistic regression, (diagonal) linear or quadratic discriminant analysis (LDA, QDA, DLDA, DQDA), perceptron, shrunken centroids regularized discriminant analysis (RDA), random forests (RF), neural networks (NN), hidden Markov models, generalized partial least squares (GPLS), partitioning around medoids (PAM), inductive logic programming (ILP), generalized additive models, gaussian processes, regularized least square regression, self organizing maps (SOM), recursive partitioning and regression trees, K-nearest neighbor classifiers (K-NN), fuzzy classifiers, bagging, boosting can be used.

Further aspects, advantages and embodiments of the present invention will become evident by the description of examples, from the experimental sections below and by means of the drawings.

As used herein, "inflammation-related brain injury in preterm born infants" includes all stages of brain inflammatory injury in preterm born infants

"Inflammatory brain injury in preterm born infants" refers to a brain injury in preterm born infants-positive condition that is associated with a confirmed inflammatory process.

The "onset of inflammatory brain injury in preterm born infants" refers to an early stage of inflammatory brain injury in preterm born infants, i.e., prior to a stage when the clinical manifestations are sufficient to support a clinical suspicion of inflammatory brain injury in preterm born infants. The exact mechanism by which a patient acquires inflammatory brain injury in preterm born infants is not a critical aspect of the invention. The methods of the present invention can detect changes in the biomarker score independent of the origin of the inflammatory brain injury in preterm born infants. Regardless of how inflammatory brain injury in preterm born infants arises, the methods of the present invention allow for determining the status of a patient having, or suspected of having, brain injury in preterm born infants, as classified by previously used criteria.

As used herein, the term "inflammatory brain injury in preterm born infants specific metabolite" refers to metabolites that are differentially present or differentially concentrated in brain-damaged organisms compared to healthy organisms.

A brain injury in preterm born infants -specific metabolite is preferably differentially present at a level that is statistically significant (e.g., an adjusted p-value less than 0.05 as determined using either Analysis of Variance, Welch's t-test or its non parametric equivalent versions). Exemplary inflammatory brain injury in preterm born infants - specific metabolites are described in the detailed description and experimental sections below.

A blood sample may be plasma or serum.

A "reference level" of a metabolite means a level of the metabolite that is indicative of a particular disease state, phenotype, or lack thereof, as well as combinations of disease states, phenotypes, or lack thereof. A "positive" reference level of a metabolite means a level that is indicative of a particular disease state or phenotype. A "negative" reference level of a metabolite means a level that is indicative of a lack of a particular disease state or phenotype. For example, an "inflammatory brain injury in preterm born infants - positive reference level" of a metabolite means a level of a metabolite that is indicative of a positive diagnosis of inflammatory brain injury in preterm born infants in a subject, and an "inflammatory brain injury in preterm born infants -negative reference level" of a metabolite means a level of a metabolite that is indicative of a negative diagnosis of brain injury in preterm born infants in a subject. A "reference level" of a metabolite may be an absolute or relative amount or concentration of the metabolite, a presence or absence of the metabolite, a range of amount or concentration of the metabolite, a minimum and/or maximum amount or concentration of the metabolite, a mean amount or concentration of the metabolite, and/or a median amount or concentration of the metabolite; and, in addition, "reference levels" of combinations of metabolites may also be ratios of absolute or relative amounts or concentrations of two or more metabolites with respect to each other or a composed value / score obtained by classification.

As used herein, the term "processor" refers to a device that performs a set of steps according to a program (e.g., a digital computer). Processors, for example, include Central Processing Units ("CPUs"), electronic devices, or systems for receiving, transmitting, storing and/or manipulating data under programmed control.

As used herein, the term "memory device," or "computer memory" refers to any data storage device that is readable by a computer, including, but not limited to, random access memory, hard disks, magnetic (floppy) disks, compact discs, DVDs, magnetic tape, flash memory, and the like.

"Mass Spectrometry" (MS) is a technique for measuring and analyzing molecules that involves fragmenting a target molecule, then analyzing the fragments, based on their mass/charge ratios, to produce a mass spectrum that serves as a "molecular fingerprint". Determining the mass/charge ratio of an object is done through means of determining the wavelengths at which electromagnetic energy is absorbed by that object. There are several commonly used methods to determine the mass to charge ratio of an ion, some measuring the interaction of the ion trajectory with electromagnetic waves, others measuring the time an ion takes to travel a given distance, or a combination of both. The data from these fragment mass measurements can be searched against databases to obtain definitive identifications of target molecules. Mass spectrometry is also widely used in other areas of chemistry, like petrochemistry or pharmaceutical quality control, among many others.

As used here, the term "metabolite" denotes endogenous organic compounds of a cell, an organism, a tissue or being present in body liquids and in extracts obtained from the aforementioned sources with a molecular weight typically below 1500 Dalton. Typical examples of metabolites are carbohydrates, lipids, phospholipids, sphingolipids and sphingophospholipids, amino acids, cholesterol, steroid hormones and oxidized sterols and other compounds such as collected in the Human Metabolite database [Wishart DS et al., HMDB: the Human Metabolome Database. Nucleic Acids Res. 2007 Jan;35 (Database issue):D521-6 (see http://www.hmdb.ca/)*]* and other databases and literature. This includes any substance produced by metabolism or by a metabolic process and any substance involved in metabolism.

"Metabolomics" as understood within the scope of the present invention designates the comprehensive quantitative measurement of several (2-thousands) metabolites by, but not limited to, methods such as mass spectroscopy, coupling of liquid chromatography, gas chromatography and other separation methods chromatography with mass spectroscopy.

The term "separation" refers to separating a complex mixture into its component proteins or metabolites. Common laboratory separation techniques include gel electrophoresis and chromatography.

The term "capillary electrophoresis" refers to an automated analytical technique that separates molecules in a solution by applying voltage across buffer-filled capillaries. Capillary electrophoresis is generally used for separating ions, which move at different speeds when the voltage is applied, depending upon the size and charge of the ions. The solutes (ions) are seen as peaks as they pass through a detector and the area of each peak is proportional to the concentration of ions in the solute, which allows quantitative determinations of the ions.

The term "chromatography" refers to a physical method of separation in which the components to be separated are distributed between two phases, one of which is stationary (stationary phase) while the other (the mobile phase) moves in a definite direction. Chromatographic output data may be used for manipulation by the present invention.

An "ion" is a charged object formed by adding electrons to or removing electrons from an atom.

A "mass spectrum" is a plot of data produced by a mass spectrometer, typically containing m/z values on x-axis and intensity values on y-axis.
A "peak" is a point on a mass spectrum with a relatively high y-value.
The term "m/z" refers to the dimensionless quantity formed by dividing the mass number of an ion by its charge number. It has long been called the "mass-to-charge" ratio.

The term "metabolism" refers to the chemical changes that occur within the tissues of an organism, including "anabolism" and "catabolism". Anabolism refers to biosynthesis or the buildup of molecules and catabolism refers to the breakdown of molecules.

As used herein, the terms "detect", "detecting", or "detection" may describe either the general act of discovering or discerning or the specific observation of a detectably labelled composition.

As used herein, the term "clinical failure" refers to a negative outcome following inflammatory brain injury in preterm born infants treatment.

A biomarker in this context is a characteristic, comprising data of at least two metabolites that is measured and evaluated as an indicator of biologic processes, pathogenic processes, or responses to a therapeutic intervention associated with inflammatory brain injury in preterm born infants or related to brain injury in preterm born infants treatment. A combined biomarker as used here may be selected from at least two small endogenous molecules and metabolites.

### DETAILED DESCRIPTION OF THE INVENTION

The present invention relates to markers of inflammatory brain injury in preterm born infants and its duration/severity. In particular embodiments, the present invention provides metabolites that are differentially present in brain injury in preterm born infants. Experiments conducted during the course of development of the embodiments of the present invention identified a series of metabolites as being differentially present in subjects in brain injury in preterm born infants in comparison to those without inflammatory brain injury in preterm born infants.

### Diagnostic Applications

In some embodiments, the present invention provides methods and compositions for diagnosing brain injury in preterm born infants, including but not limited to, characterizing risk of brain injury in preterm born infants, stage of brain injury in preterm born infants, duration and severity etc. based on the presence of brain injury in preterm born infants specific metabolites or their derivatives, precursors, metabolites, etc. Exemplary diagnostic methods are described below.

Thus, for example, a method of diagnosing (or aiding in diagnosing) whether a subject has inflammatory brain injury in preterm born infants comprises (1) detecting the presence or absence or a differential level of a plurality of metabolites being specific for brain injury in preterm born infants, such specific metabolites are the following five compounds of a selection at time point 216h: Phenylthiocarbamyltyrosine, Lysophosphatidylcholine with acyl residue C28:1, Phenylalanine, Methionine sulfoxide and Putrescine and b) diagnosing inflammatory brain injury in preterm born infants based on the presence, absence or differential level of the brain injury in preterm born infants specific metabolites.

In some embodiments, a computer-based analysis program is used to translate the raw data generated by the detection assay (e.g., the presence, absence, or amount of a brain injury in preterm born infants specific metabolite) into data of predictive value for a clinician. The clinician can access the predictive data using any suitable means. Thus, in some embodiments, the present invention provides the further benefit that the clinician, who is not likely to be trained in metabolite analysis, need not understand the raw data. The data is presented directly to the clinician in its most useful form. The clinician is then able to immediately utilize the information in order to optimize the care of the subject.

The present invention contemplates any method capable of receiving, processing, and transmitting the information to and from laboratories conducting the assays, information provides, medical personal, and subjects. The profile data is then prepared in a format suitable for interpretation by a treating clinician. For example, rather than providing raw data, the prepared format may represent a diagnosis or risk assessment (e.g., likelihood of inflammatory brain injury in preterm born infants being present) for the subject, along with recommendations for particular treatment options. The data may be displayed to the clinician by any suitable method. For example, in some embodiments, the profiling service generates a report that can be printed for the clinician (e.g., at the point of care) or displayed to the clinician on a computer monitor.

In some embodiments, the information is first analyzed at the point of care or at a regional facility. The raw data is then sent to a central processing facility for further analysis and/or to convert the raw data to information useful for a clinician or patient. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the clinician, the subject, or researchers.

When the amounts or levels of a plurality of metabolites in the sample are determined, the amounts or levels may be compared to brain injury in preterm born infants metabolite-reference levels, such as - inflammatory brain injury in preterm born infants - positive and/or inflammatory brain injury in preterm born infants -negative reference levels to aid in diagnosing or to diagnose whether the subject has brain injury in preterm born infants. Levels of the plurality of metabolites in a sample corresponding to the inflammatory brain injury in preterm born infants -positive reference levels (e.g., levels that are the same as the reference levels, substantially the same as the reference levels, above and/or below the minimum and/or maximum of the reference levels, and/or within the range of the reference levels) are indicative of a diagnosis of inflammatory brain injury in preterm born infants in the subject.

In addition, levels of a plurality metabolites that are differentially present (especially at a level that is statistically significant) in the sample as compared to inflammatory brain injury in preterm born infants-negative reference levels are indicative of a diagnosis of brain injury in preterm born infants in the subject. Levels of the two or more metabolites that are differentially present (especially at a level that is statistically significant) in the sample as compared to inflammatory brain injury in preterm born infants -positive reference levels are indicative of a diagnosis of no inflammatory brain injury in preterm born infants in the subject.

The level(s) of a plurality of the metabolites may be compared to inflammatory brain injury in preterm born infants -positive and/or inflammatory brain injury in preterm born infants -negative reference levels using various techniques, including a simple comparison (e.g., a manual comparison) of the level(s) of a set of metabolites in the sample to brain injury in preterm born infants -positive and/or brain injury in preterm born infants -negative reference levels. The level(s) of the set of metabolites in the blood sample may also be compared to inflammatory brain injury in preterm born infants and/or inflammatory brain injury in preterm born infants -negative reference levels using one or more statistical analyses (e.g., t-test, Welch's t-test, Wilcoxon's rank sum test, random forests, linear discriminant analysis, k nearest neighbours).

In some embodiments, panel or combination assays are provided that detected 5 or more, 6 or more, 7 or more, 8 or more, 9 or more, 10 or more markers in a single assay. In some embodiments, assays are automated or high throughput.

A preferred embodiment of the present invention is the use of the following five markers of a selection at time point 216h: Phenylthiocarbamyltyrosine, Lysophosphatidylcholine with acyl residue C28:1, Phenylalanine, Methionine sulfoxide and Putrescine for diagnosis of inflammatory brain injury in preterm born infants and its duration/severity where said mammalian subject is a human being.

### EXPERIMENTAL CONDITIONS

The following examples are provided in order to demonstrate and further illustrate certain preferred embodiments and aspects of the present invention and are not to be construed as limiting the scope thereof.

### General Analytics:

Sample preparation and metabolomic analyses were performed at BIOCRATES life sciences AG, Innsbruck, Austria. We used a multi-parametric, highly robust, sensitive and high-throughput targeted metabolomic platform consisting of flow injection analysis (FIA)-MS/MS and LC-MS/MS methods for the simultaneous quantification of a broad range of endogenous intermediates namely from the panel disclosed in table1. All procedures (sample handling, analytics) were performed by co-workers blinded to the groups.

### Sample handling

### Plasma

Plasma samples were prepared by standard procedures and stored at (-75°C). To enable analysis of all samples simultaneously within one batch, samples were thawed on ice (1 h) on the day of analysis and centrifuged at 18000 g at 2°C for 5 min. All tubes were prepared with 0.001% BHT (butylated hydroxytoluene; Sigma-Aldrich, Vienna, Austria) to prevent autooxidation.

### LC-MS/MS system

The LC-MS/MS system consisted of an API 4000™ triple quadrupole mass spectrometer (AB Sciex) equipped with a Turbo V™ ESI source and an Agilent 1200 hplc system (Agilent Technologies). Chromatographic separation was performed using an Agilent Zorbax Eclipse XDB C18 column (100 x 3.0 mm, 3.5 µm) with guard column (C 18, 4 x 2 mm in Security Guard Cartridge, Phenomenex). Analyst™ software (version 1.4.2, Applied Biosystems) was used for data acquisition and processing. For comprehensive statistical analysis the data were exported.

### LC-MS/MS conditions

The ESI source was operated in negative ion mode and an ion-spray voltage of -3 kV was applied. Heater temperature was set at 400 °C.

### Detailed Examples

Fetal metabolic response to intrauterine inflammation in terms of changes of metabolite concentrations is detected in preterm sheep; intrauterine inflammation is known to cause brain injury.

We used a well established animal model of intrauterine inflammation in fetal sheep.

### Experimental procedures

We employed a model of LPS-induced brain injury in fetal sheep as previously described (Svedin 2005, Blad, 2008 and Dean 2009).

### Surgery

Fetal surgery was performed as previously described (Mallard 2003). Specifically, food was withdrawn 12 hours before the operation to avoid complications during anaesthesia. Pre-mediation on the day of surgery was Stesolid (0.1-0.2mg/kg, iv) and Temgesic (0.005-0.02 mg/kg, iv). Anaesthesia was induced by Pentothal Sodium followed by intubation and Isofluoran (1.5%) anaesthesia.

The uterine horn was exposed through a midline incision and the fetal head was identified and fetal biparietal diameter was measured. A small hysterectomy incision was made over the fetal head through the uterine wall, parallel to any vessels. Polyvinyl catheters (i.d. 1 mm, Smiths Medical) were inserted into each brachial/axilliary artery and one brachial vein. An amnion catheter (i.d. 2.0 mm, portex, Smiths Medical) was secured to one of the ears. The fetal scalp overlying the parasagittal cortex was exposed and two bilateral holes drilled through the skull but avoiding the dura at 5 and 15 mm anterior of bregma and 0.5 mm lateral of midline. Two pairs of EEG electrodes (P/N Ag7/40T, Leico Ind) were inserted through the burr holes and secured to the skull with a small rubber disk glued with cyanoacrylate and skin flaps were glued back over the electrodes. A reference electrode was placed subcutaneously anterior to the EEG electrodes and one ground electrode subcutaneously in the neck. One additional reference electrode was placed on the ear.

At the end of the operation, catheters were filled with 50E/ml heparin. The uterus was closed in two layers and catheters and electrodes exteriorized via troakar. One vein catheter was placed in the tarsal vein of the ewe. In case of twins, only one was is instrumented.

The surgery length was approximately 2 hours. Following surgery, the ewe was housed in a metabolic cage with free access to food and water. A minimum of 4 days was allowed for recovery from surgery before any studies were commenced. During this recovery period, fetal arterial blood was sampled daily to monitor fetal oxygenation, glucose and lactate status.

### Experimental protocol

Animals were randomly assigned to *Escherichia coli* Lipopolysaccharide (LPS, Sigma 055:B4, 200ng/fetus) exposure or sham control group. Fetal EEG (BrainZ), fetal arterial pressure and amniotic pressure (Biopac) was continuously recorded on-line from a minimum of 6 hours before LPS exposure until 10 days after. Blood and amniotic samples were collected on ice according to Biocrates operation procedures. Blood was sampled at the following time points: 10 min before LPS and 2h, 6h and 24h after LPS and then once daily until sacrifice. The experiments were terminated 10 days after LPS bolus by an i.v. infusion of Pentobarbital to the maternal vein.

### Post mortem

Following termination of the experiment, the fetus was immediately removed from the uterus, weighed and sexed and tissue samples (liver, heart, adrenals, kidney, spleen) were collected on isopentane/ice and stored at -80C. Following tissue collection, the fetal brain was perfused in situ (through catheters into a. carotis) with 0.9% NaCl (500ml) followed by 500 ml of 4% paraformaldehyde (Histofix). The brain was removed and stored in fix for at least 24h, followed by MRI and histology analysis to confirm grey matter brain injury

### Mass spectroscopy

We used a multiparametric, highly robust, sensitive and high-throughput targeted metabolomic LC-MS/MS method for the simultaneous quantification of endogenous intermediates (amino acids, biogenic amines, acylcarnitines, sphingomyelins and glycerophospholipids, eicosanoides, oxysterols) in plasma samples enabling the determination of a broad range of analytes. All assays/ analytical methods are validated according the FDA guidance for bioanalytical methods for human plasma.

### Acylcarnitines, Amino acids, Sphingomyelins, Glycerophosphatidylcholines (FIA-MS/MS)

To determine the concentration of acylcarnitines, sphingomyelins and glycerophospholipids the AbsolutelDQ kit p150 (BIOCRATES Life Sciences AG, Innsbruck, Austria) was prepared as described in the manufacturer's protocol. In brief, 10 µl sample was added to the center of the filter on the upper 96-well kit plate, and the samples were dried using a nitrogen evaporator (VLM Laboratories, Leopoldshöhe, Germany). The metabolites were extracted using 300 µl of a 5 mM ammonium acetate solution in methanol. The extracts were obtained by centrifugation into the lower 96-deep well plate followed by a dilution step with 600 µl of kit MS running solvent. Mass spectrometric analysis was performed on a 4000 Q Trap® tandem mass spectrometry instrument (Applied Biosystems/MDS Analytical Technologies, Darmstadt, Germany) equipped with a Turbo V™ electrospray ionization (ESI) source using the analysis acquisition method as provided in the AbsoluteIDQ kit. The standard FIA-MS/MS method was applied for all measurements with two subsequent 20 µl injections (one for positive and one for negative mode analysis). Multiple reaction monitoring (MRM) detection was used for quantification applying the spectra parsing algorithm integrated into the MetIQ software (BIOCRATES Life Sciences AG, Innsbruck, Austria).

### Amino acids, Biogenic amines (LC-MS/MS)

Amino acids and biogenic amines were quantitatively analyzed by reversed phase LC-MS/MS to obtain chromatographic separation of isobaric (same MRM ion pairs) metabolites for individual quantitation performed by external calibration and by use of internal standards. 10 µl sample volume is required for the analysis using the following sample preparation procedure. Samples were added on filter spots placed in a 96-solvinert well plate (internal standards were placed and dried down under nitrogen before), fixed above a 96-deep well plate (capture plate). 20 µl of 5 % phenyl-isothiocyanate derivatization reagent was added. The derivatized samples were extracted after incubation by aqueous methanol into the capture plate. 10 µl sample extracts were analyzed by LC-ESI-MS/MS in positive MRM detection mode with a 4000 Q Trap® tandem mass spectrometry instrument (Applied Biosystems/MDS Analytical Technologies, Darmstadt, Germany).

### Energy metabolism (Organic Acids) (LC-MS/MS)

For the quantitative analysis of energy metabolism intermediates (glycolysis, citrate cycle, pentose phosphate pathway, urea cycle) hydrophilic interaction liquid chromatography HILIC-ESI-MS/MS method in highly selective negative MRM detection mode was used. The MRM detection was performed using API400 QTRAP tandem mass spectrometry instrument (Applied Biosystems/MDS Analytical Technologies). 20 µL sample volume was protein precipitated and extracted simultaneously with aqueous methanol in a 96-well plate format. Internal standards (ratio external to internal standard)and external calibration were used for highly accurate quantitation. Data were quantified with Analyst 1.4.2 software (applied Biosystems) and finally exported for statistical analysis.

Samples were stored at - 80° C for subsequent assaying.

### RESULTS

Table1: LPS injection induced a significant time dependent change in the fetal sheep plasma metabolome including but not limited to markers of immune response, energy metabolism as well as tissue injury. Thereby 22 metabolites significantly correlated with the aEEG pattern at 192 hours (p<0,05, r2 = 0,8- 0,947) and 5-18 metabolites at 216 and 240h with histological assessed grey and white matter volume (p<0,05, r2 = 0,8-0,947).

### List of metabolite biomarkers:

### Outcome parameter: aEEG at 192h

| **Company ID** | **Compound** | **CAS** | **p-values** | **correlations** | **R^2** |
|---|---|---|---|---|---|
| C0 | Carnitine (free) | 461-06-3 | 0.01 | 0.916 | 0.838 |
| C12-DC | Dodecanedioylcarnitine | | 0.004 | 0.947 | 0.897 |
| C3 | Propionylcarnitine | 17298-37-2 | 0.008 | 0.926 | 0.858 |
| C3:1 | Propenoylcarnitine | | 0.043 | 0.825 | 0.68 |
| C4 | Butyrylcarnitine / Isobutyrylcarnitine | 25576-40-3 /25518-49-4 | 0.032 | 0.849 | 0.721 |
| C4:1 | Butenoylcarnitine | | 0.012 | 0.911 | 0.83 |
| C5 | Isovalerylcarnitine / 2-Methylbutyrylcarnitine / Valerylcarnitine | 31023-24-2 | 0.049 | 0.812 | 0.66 |
| C5:1 | Glutaconylcarnitine / Mesaconylcarnitine | | 0.049 | 0.813 | 0.662 |
| PC aa C28:1 | Phosphatidylcholine with diacyl residue sum C28:1* | | 0.033 | 0.847 | 0.717 |
| PC aa C30:0 | Phosphatidylcholine with diacyl residue sum C30:0 | | 0.043 | 0.826 | 0.682 |
| PCaa C32:2, | Phosphatidylcholine with diacyl residue sum C32:2 | | 0.028 | 0.861 | 0.741 |
| PC aa C34:2 | Phosphatidylcholine with diacyl residue sum C34:2 | | 0.032 | 0.851 | 0.724 |
| PC aa C34:3 | Phosphatidylcholine with diacyl residue sum C34:3 | | 0.019 | 0.886 | 0.785 |
| PC aa C34:4 | Phosphatidylcholine with diacyl residue sum C34:4 | | 0.029 | 0.857 | 0.734 |
| PC aa C36:2 | Phosphatidylcholine with diacyl residue sum C36:2 | | 0.019 | 0.885 | 0.783 |
| PC aa, C40:2 | Phosphatidylcholine with diacyl residue sum C40:2 | | 0.043 | 0.825 | 0.681 |
| PC ae C30:1 | Phosphatidylcholine with acyl-alkyl residue sum C30:1 | | 0.015 | 0.898 | 0.807 |
| PC ae C36:2 | Phosphatidylcholine with acyl-alkyl residue sum C36:2 | | 0.047 | 0.817 | 0.668 |
| PC ae C38:2 | Phosphatidylcholine with acyl-alkyl residue sum C38:2 | | 0.02 | 0.882 | 0.778 |
| PC ae C40:2 | Phosphatidylcholine with | | 0.047 | 0.818 | 0.669 |
| | acyl-alkyl residue sum C40:2 | | | | |
| Trp | Tryptophane | 73-22-3 | 0.019 | -0.884 | 0.782 |
| Kyn | Kynurenine | 343-65-7 | 0.043 | 0.825 | 0.681 |

| | | | | | |
|---|---|---|---|---|---|
| *wherein the number following "C" represents the number of carbon atoms in the residue, and the number after the colon represents the number of double bonds in the residue. | | | | | |

### Outcome parameter: GM volume - Time point 240h:

| **Company ID** | **Compound** | **CAS** | **p-values** | **Correlations** | **R^2** |
|---|---|---|---|---|---|
| C0 | Carnitine (free) | 461-06-3 | 0.038 | 0.899 | 0.808 |
| C14:1 | Tetradecenoylcarnitine [Myristoleylcarnitine] | | 0.048 | 0.881 | 0.777 |
| C3 | Propionylcarnitine | 17298-37-2 | 0.04 | 0.896 | 0.803 |
| C6 (C4:1-DC) | Hexanoylcarnitine [Caproylcarnitine] | 14919-34-7 | 0.042 | 0.892 | 0.796 |
| PC aa C36:0 | Phosphatidylcholine with diacyl residue sum C36:0* | | 0.022 | 0.93 | 0.864 |
| PC aa C36:1 | Phosphatidylcholine with diacyl residue sum C36:1 | | 0.012 | 0.954 | 0.909 |
| PC aa C38:3 | Phosphatidylcholine with diacyl residue sum C38:3 | | 0.04 | 0.896 | 0.802 |
| PC aa C38:6 | Phosphatidylcholine with diacyl residue sum C38:6 | | 0.045 | 0.886 | 0.785 |
| PC aa C40:5 | Phosphatidylcholine with diacyl residue sum C40:5 | | 0.05 | 0.879 | 0.772 |
| PC aa C40:6 | Phosphatidylcholine with diacyl residue sum C40:6 | | 0.02 | 0.934 | 0.873 |
| PC ae C36:1 | Phosphatidylcholine with acyl-alkyl residue sum C36:1 | | 0.023 | 0.928 | 0.86 |
| PC ae C38:1 | Phosphatidylcholine with acyl-alkyl residue sum C38:1 | | 0.043 | 0.89 | 0.792 |
| ADMA | Asymmetric dimethylarginine | | 0.038 | 0.898 | 0.807 |
| SDMA | Symmetric dimethylarginine | | 0.035 | 0.904 | 0.817 |
| total DMA | Total dimethylarginine | | 0.041 | 0.894 | 0.799 |

| | | | | | |
|---|---|---|---|---|---|
| *wherein the number following "C" represents the number of carbon atoms in the residue, and the number after the colon represents the number of double bonds in the residue. | | | | | |

### Outcome parameter WM volume

### - time point 216h:

| **Biocrates ID** | **Compound** | **CAS** | **p-value** | **correlations** | **R^2** |
|---|---|---|---|---|---|
| Met-PTC** | Phenylthiocarbamyl-methionine | (63-68-3) | 0,041 | -0.831 | 0.69 |
| Phe-PTC* | Phenylthiocarbamyl-phenylalanine | (63-91-2) | 0.032 | -0.849 | 0.721 |
| Ser-PTC* | Phenylthiocarbamyl-serine | (56-45-1) | 0.033 | -0.847 | 0.718 |
| Tyr-PTC* | Phenylthiocarbamyl-tyrosine | (60-18-4) | 0.01 | -0.918 | 0.842 |
| C16-OH | 3-Hydroxyhexadecanol ycarnitine [3-Hydroxypalmitoylcar nitine] | | 0.04 | -0.832 | 0.692 |
| PC aa C24:0 | Phosphatidylcholine with diacyl residue sum C24:0* | | 0.042 | -0.829 | 0.687 |
| IysoPC a C28:1 | Lysophosphatidylcho line with acyl residue C28:1 | | 0.006 | -0.938 | 0.881 |
| Ser | Serine | 56-45-1 | 0.043 | 0.826 | 0.682 |
| Pro | Proline | 147-85-3 | 0.049 | -0.814 | 0.663 |
| Val | Valine | 72-18-4 | 0.049 | -0.813 | 0.661 |
| Phe | Phenylalanine | 63-91-2 | 0.009 | -0.922 | 0.851 |
| Cit | Citrulline | 372-75-8 | 0.019 | -0.885 | 0.783 |
| Tyr | Tyrosine | 60-18-4 | 016 | -0.894 | 0.8 |
| Met-SO | Methionine sulfoxide | 62697-73-8 | 0.01 | -0.917 | 0.841 |
| Putrescine | Putrescine | 110-60-1 | 0.012 | -0.909 | 0.826 |
| Lac | Lactate | 50-21-5 | 0.035 | 0.844 | 0.713 |

| | | | | | |
|---|---|---|---|---|---|
| *wherein the number following "C" represents the number of carbon atoms in the residue, and the number after the colon represents the number of double bonds in the residue. **Amino acids are detected as phenylthiocarbamyl-derivate after phenylisothiocyanate derivatization. | | | | | |

### - Time point 240h:

| **Biocrates ID** | **Compound** | **CAS** | **p-value** | **correlations** | **R^2** |
|---|---|---|---|---|---|
| Gly-PTC* | Phenylthiocarbamyl-glycine | (56-40-6) | 0.049 | | |
| C0 | Carnitine (free) | 461-06-3 | 0.013 | | |
| C2 | Acetylcarnitine | 3040-38-8 | 0.043 | | |
| C3 | Propionylcarnitine | 17298-37-2 | 0.006 | | |
| C4 | Butyrylcarnitine / Isobutyrylcarnitine | 25576-40-3 /25518-49-4 | 0.044 | | |
| C5 | Isovalerylcarnitine / 2-Methylbutyrylcarnitine / Valerylcarnitine | 31023-24-2 | 0.049 | | |
| C5-DC (C6-OH) | Glutarylcarnitine | | 0.041 | | |
| C5:1 | Tiglylcarnitine / 3-Methyl-crotonylcarnitine | | 0.049 | | |
| PC aa C36:0 | Phosphatidylcholine with diacyl residue sum C36:0* | | 0.013 | | |
| PC aa, C36:1 | Phosphatidylcholine with diacyl residue sum C36:1 | | 0.012 | | |
| PC ae C36:1 | Phosphatidylcholine with acyl-alkyl residue sum C36:1 | | 0.03 | | |
| Gly | Glycine | 56-40-6 | 0.041 | | |
| ADMA | Asymmetric dimethylarainine | | 0.033 | | |
| SDMA | Symmetric dimethylarginine | | 0.005 | | |
| total DMA | Total dimethylarginine | | 0.017 | | |
| Lac | Lactate | 50-21-5 | 0.027 | | |

| | | | | | |
|---|---|---|---|---|---|
| *wherein the number following "C" represents the number of carbon atoms in the residue, and the number after the colon represents the number of double bonds in the residue **Amino acid is detected as phenyltiocarbamyl-derivate after phenyl-isothiocyanate derivatization | | | | | |

In a second step we used all metabolites to compute multivariate models. As classification algorithms we employed random forests (RF), k-nearest neighbors (KNN), and linear discriminant analysis (LDA). The precision of the prediction was assessed by bootstrap resampling where 50 replications were performed. Tab. 2 includes the corresponding results

**Table 2: Classification of samples into hypoxic and control groups across all sample timepoints. AUC (area under the curve), accuracy, sensitivity and specificity for various classifiers (RF = Random Forest, LDA = Linear Discriminant Analysis, KNN = K Nearest Neighbors). The numbers in brackets are the results for bootstrap resampling.**

| Classifier | AUC | Accuracy | Sensitivity | Specificity |
|---|---|---|---|---|
| RF | 0.93 (0.84) | 0,92 (0.77) | 1.00 (0.89) | 0.75 (0.4) |
| LDA | 0.81 (0.61) | 0.77 (0.62) | 0,78 (0.68) | 0.75 (0.58) |
| KNN | 0.83 (0.72) | 0.84 (0.77) | 1.00 (0.88) | 0.50 (0.67) |

**Table 3: Classification results of LPS and sham treated sheep samples taken at late time points (216h and 240h). AUC (area under the curve), accuracy, sensitivity and specificity for various classifiers (RF = Random Forest, LDA = Linear Discriminant Analysis, KNN = K Nearest Neighbors)The classification models are based on the best five markers of the selection at time point 216h. The values in brackets refer to classification with 50 bootstrap resampling runs.**

| Classifier | AUC | Accuracy | Sensitivity | Specificity |
|---|---|---|---|---|
| RF | 0.83 (0.75) | 1.00 (0.73) | 1.00 (0.67) | 1.00 (0.75) |
| LDA | 0.78 (0.71) | 0.83 (0.82) | 1.00 (0.83) | 0.67 (1.00) |
| KNN | 0.83 (0.61) | 0.83 (0.67) | 1.00 (0.71) | 0.67 (0.50) |

The classification is based upon such compounds exhibiting the best p-values from the table for samples for time point 216 h. According to the classification of Table 3, an accuracy of greater 70% can be achieved by using only 5 compounds as a subset of biomarkers. In other words, with a subset of only 5 compounds, in accordance with the present invention, it is possible to predict the likelihood of inflammation-related brain injury in preterm born infants with an accuracy of greater than 70%.

Even better results can be achieved by choosing more than 5, in particular at least 8, at least 10, at least 12, at least 15, or at least 18 compounds according to claims 1 or 12.

### Statistical Analysis

All statistical calculations have been performed using the statistics software R (R: A Language and Environment for Statistical Computing, R Development Core Team, R Foundation for Statistical Computing, Vienna, Austria, 2010, ISBN 3-900051-07-0).

All analytes that were detected in at least 15% of the samples were selected for further analyses. The metabolic data is left censored due to thresholding of the mass spectrometer data resulting in non detected peak/signals. By a combination of metabolic pathway dynamism, complex sample molecular interaction and overall efficiency of the analytical protocol, replacement of missing data by means of a multivariate algorithm is preferred to a naive imputation by a pre-specified value like for instance zero. Hence, missing metabolite concentrations are replaced by the average value of the 6 closest samples to the one where the measurement is missing (impute: Imputation for microarray data, Hastie T., Tibshirani R., Narasimhan B. and Chu G., R package version 1.14.0). At the exception of fold change (FC) determination, all statistical analyses are performed on preprocessed - that is, log transformed - data. The log-transformation is used to stabilize variance and to transform to Gaussian distribution - at least approximately.

The ImFit function in the package limma (limma: linear models for microarray data, Smyth G.K. In: Bioinformatics and Computational Biology Solutions using R and Bioconductor, Springer, New York, pp 397-420, R package version 2.16.5) is used to compute the moderated statistics for pair wise comparisons between measurements from control samples and samples with brain injury in preterm born infants. Resulting p values are adjusted by the method described in Benjamini and Hochberg (Benjamini Y. and Hochberg Y., Controlling the false discovery rate: a practical and powerful approach to multiple testing, Journal of the Royal Statistical Society Series B, 1995, 57, 289-300) leading to so-called q values.

Sensitivity/specificity properties of a classifier comprising one analyte or a combination of analytes are summarised in terms of Area Under the Receiver Operating Characteristic Curve (AUC). The function colAUC (caTools: Tools: moving window statistics, GIF, Base64, ROC AUC, etc., Tuszynski J., 2008, R package version 1.9) is used to compute AUC values.

A higher reproducibility of the results can be achieved by combining criteria like log2-FC, p resp. q value and AUC.

Multivariate classifications based on the full set of metabolites were calculated by the use of Random Forest as implementation in the R-package randomForest (Classification and Regression by randomForest, Liaw A. and Wiener M., R News 2002, 2:3, 18-22), Linear Discriminant Analysis from the R-package sfsmisc (sfsmisc: Utilities from Seminar fuer Statistik ETH Zurich, Maechler M. and many others, R package version 1.0-13) and K Nearest Neighbours from the package caret (caret: Classification and regression Training, Kuhn M., 2010, R package version 4.72). The Classification is based on the metabolite panel that was jointly measured for all samples.

## Claims

1. A method for predicting the likelihood of inflammation-related brain injury in preterm born infants, with a greater than 70% accuracy,
**characterized by**
quantitatively detecting in vitro in at least one blood sample of a patient a plurality of at least the following five compounds of a selection at time point 216h:
Phenylthiocarbamyltyrosine, Lysophosphatidylcholine with acyl residue C28:1, wherein the number following "C" in the Lysophosphatidylcholine represents the number of carbon atoms in the residue, and the number after the colon represents the number of double bonds in the residue, Phenylalanine, Methionine sulfoxide and Putrescine, and being specific for inflammation-related brain injury, and having a molecular weight of less than 1500 Dalton comprising the steps of:
a) selecting further compounds from an endogenous target metabolite group consisting of: Acetylcarnitine; Dodecanedioylcarnitine; Propionylcarnitine; Propenoylcarnitine; Butyrylcarnitine/Isobutyrylcarnitine; Butenoylcarnitine; Isovalerylcarnitine / 2-Methylbutyrylcarnitine / Valerylcarnitine; Glutaconylcarnitine / Mesaconylcarnitine; Glutarylcarnitine; Tetradecenoylcarnitine; 3-Hydroxyhexadecanoyl carnitine; Triglylcarnitine/ 3-Methyl-crotonylcarnitine; Phosphatidylcholine with diacyl residue sum C24:0; Phosphatidylcholine with diacyl residue sum C28:1; Phosphatidylcholine with diacyl residue sum C30:0; Phosphatidylcholine with diacyl residue sum C32:2; Phosphatidylcholine with diacyl residue sum C34:2; Phosphatidylcholine with diacyl residue sum C34:3; Phosphatidylcholine with diacyl residue sum C34:4; Phosphatidylcholine with diacyl residue sum C36:0; Phosphatidylcholine with diacyl residue sum C36:1; Phosphatidylcholine with diacyl residue sum C36:2; Phosphatidylcholine with diacyl residue sum C38:3; Phosphatidylcholine with diacyl residue sum C38:6; Phosphatidylcholine with diacyl residue sum C40:2; Phosphatidylcholine with diacyl residue sum C40:5; Phosphatidylcholine with diacyl residue sum C40:6; Phosphatidylcholine with acyl-alkyl residue sum C30:1; Phosphatidylcholine with acyl-alkyl residue sum C36:1; Phosphatidylcholine with acyl-alkyl residue sum C36:2; Phosphatidylcholine with acyl-alkyl residue sum C38:1; Phosphatidylcholine with acyl-alkyl residue sum C38:2; Phosphatidylcholine with acyl-alkyl residue sum C40:2; Tryptophane; Kynurenine; asymmetric dimethylarginine; symmetric dimethylarginine; total dimethylarginine; Phenylthiocarbamyl-methionine; Phenylthiocarbamyl-phenylalanine; Phenylthiocarbamyl-serine; Phenylthiocarbamyl-glycine; Glycine; Serine; Proline; Valine; Tyrosine, Citrulline;
b) measuring at least one of the parameters selected from the group consisting of: concentration, level or amount of each specific compound of said plurality of compounds in said sample, qualitative and/or quantitative molecular pattern and/or molecular signature; and storing the obtained set of values in a database;
c) calibrating said values by comparing clinically confirmed inflammation-related brain injury in preterm born infants-positive and/or clinically confirmed inflammation-related brain injury in preterm born infants-negative reference parameters; and
d) comparing said measured values in the sample with the calibrated values, in order to assess whether the preterm neonate patient is likely to develop an inflammation-related brain injury or is unlikely to develop an inflammation-related brain injury.

2. Method according to claim 1, wherein inflammation-related brain injury comprises infection associated brain injury and/or sepsis associated brain injury.

3. Method according to claim 1 or 2, wherein said quantitative detection comprises establishing of a metabolomics profile which is achieved by a quantitative metabolomics profile analysis method comprising the generation of intensity data for the quantitation of said endogenous metabolites by mass spectrometry (MS), in particular, by high-throughput mass spectrometry, preferably by MS-technologies such as Matrix Assisted Laser Desorption/Ionisation (MALDI), Electro Spray Ionization (ESI), Atmospheric Pressure Chemical Ionization (APCI), ¹H-, ¹³C- and/or ³¹P- Nuclear Magnetic Resonance spectroscopy (NMR), optionally coupled to MS, determination of metabolite concentrations by use of MS-technologies and/or methods coupled to separation, in particular Liquid Chromatography (LC-MS), Gas Chromatography (GC-MS), or Capillary Electrophoresis (CE-MS).

4. Method according to anyone of claims 1 to 3, wherein intensity data of said metabolomics profile are normalized with a set of endogenous housekeeper metabolites by relating detected intensities of the selected endogenous target metabolites being predictive for an inflammation-related brain injury to intensities of said endogenous housekeeper metabolites.

5. Method according to claim 4, wherein said endogenous housekeeper metabolites are selected from the group consisting of such endogeneous metabolites which show stability in accordance with statistical stability measures being selected from the group consisting of coefficient of variation (CV) of raw intensity data, standard deviation (SD) of logarithmic intensity data, stability measure (M) of geNorm - algorithm or stability measure value (rho) of NormFinder-algorithm.

6. Method according to anyone of claims 1 to 5, wherein a panel of reference endogenous predictive target metabolites or derivatives thereof is established by:
a) mathematically preprocessing intensity values obtained for generating the metabolomics profiles in order to reduce technical errors being inherent to the measuring procedures used to generate the metabolomics profiles;
b) selecting at least one suitable classifying algorithm from the group consisting of logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), inductive logic programming (ILP), generalized additive models, gaussian processes, regularized least square regression, self organizing maps (SOM), recursive partitioning and regression trees, K-nearest neighbour classifiers (K-NN), fuzzy classifiers, bagging, boosting, and naïve Bayes; and applying said selected classifier algorithm to said preprocessed data of step a);
c) said classifier algorithms of step b) being trained on at least one training data set containing preprocessed data from subjects being divided into classes according to their likelihood to develop an inflammation-related brain injury, in order to select a classifier function to map said preprocessed data to said likelihood;
d) applying said trained classifier algorithms of step c) to a preprocessed data set of a subject with unknown likelihood of inflammation-related brain injury, and using the trained classifier algorithms to predict the class label of said data set in order to predict the likelihood for a subject to develop an inflammation-related brain injury.

7. Method according to anyone of claims 1 to 6, wherein said endogenous predictive target metabolites for easier and/or more sensitive detection are detected by means of chemically modified derivatives thereof, such as phenylisothiocyanates for amino acids.

8. Method according to anyone of claims 1 to 7, wherein said plurality of endogenous predictive target metabolites or derivatives thereof comprises 3 to 55, in particular 3 to 40, preferably 3 to 30, preferred 3 to 25, more preferred 3 to 22, particularly preferred 5 to 40 endogenous target metabolites.

9. Method according to anyone of claims 1 to 8, wherein lactate and/or free carnitine is/are used as additional target metabolite(s).

10. Method according to anyone of claims 1 to 9, wherein a metabolomics profile of said endogenous metabolites' group of compounds is correlated with amplitude integrated electroencephalogram (aEEG) and/or with grey matter volume and/or with white matter volume.

11. Use of a plurality of at least the following five endogenous target metabolites of the selection at time point 216h: Phenylthiocarbamyltyrosine,
Lysophosphatidylcholine with acyl residue C28:1, wherein the number following "C" in the Lysophosphatidylcholine represents the number of carbon atoms in the residue, and the number after the colon represents the number of double bonds in the residue, Phenylalanine, Methionine sulfoxide and Putrescine,
for predicting the likelihood of an onset of an inflammation-associated brain injury in preterm born infants with a greater than 70% accuracy, from a blood sample in vitro, wherein further metabolites are selected from the group consisting of : Acetylcarnitine; Dodecanedioylcarnitine; Propionylcarnitine; Propenoylcarnitine; Butyrylcarnitine/lsobutyrylcarnitine; Butenoylcarnitine; Isovalerylcarnitine / 2-Methylbutyrylcarnitine / Valerylcarnitine; Glutaconylcarnitine / Mesaconylcarnitine; Glutarylcarnitine; Tetradecenoylcarnitine; 3-Hydroxyhexadecanoyl carnitine; Triglylcarnitine/ 3-Methyl-crotonylcarnitine; Phosphatidylcholine with diacyl residue sum C24:0; Phosphatidylcholine with diacyl residue sum C28:1; Phosphatidylcholine with diacyl residue sum C30:0; Phosphatidylcholine with diacyl residue sum C32:2; Phosphatidylcholine with diacyl residue sum C34:2; Phosphatidylcholine with diacyl residue sum C34:3; Phosphatidylcholine with diacyl residue sum C34:4; Phosphatidylcholine with diacyl residue sum C36:0; Phosphatidylcholine with diacyl residue sum C36:1; Phosphatidylcholine with diacyl residue sum C36:2; Phosphatidylcholine with diacyl residue sum C38:3; Phosphatidylcholine with diacyl residue sum C38:6; Phosphatidylcholine with diacyl residue sum C40:2; Phosphatidylcholine with diacyl residue sum C40:5; Phosphatidylcholine with diacyl residue sum C40:6; Phosphatidylcholine with acyl-alkyl residue sum C30:1; Phosphatidylcholine with acyl-alkyl residue sum C36:1; Phosphatidylcholine with acyl-alkyl residue sum C36:2; Phosphatidylcholine with acyl-alkyl residue sum C38:1; Phosphatidylcholine with acyl-alkyl residue sum C38:2; Phosphatidylcholine with acyl-alkyl residue sum C40:2; Tryptophane; Kynurenine; asymmetric dimethylarginine; symmetric dimethylarginine; total dimethylarginine; Phenylthiocarbamyl-methionine; Phenylthiocarbamyl-phenylalanine; Phenylthiocarbamyl-serine; Phenylthiocarbamyl-glycine; Glycine; Serine; Proline; Valine; Tyrosine, Citrulline.

12. Use according to claim 11, wherein lactate and/or free carnitine is/are used as additional metabolite(s)

13. Use according to claim 11 or 12, wherein the sample is blood plasma.

## Patentansprüche

1. Ein Verfahren zur Vorhersage der Wahrscheinlichkeit einer entzündungsbedingten Hirnschädigung bei Frühgeborenen mit einer Genauigkeit von mehr als 70%,
**gekennzeichnet durch**
quantitativer Nachweis in vitro in mindestens einer Blutprobe eines Patienten einer Vielzahl von mindestens den folgenden fünf Verbindungen aus einer Auswahl zum Zeitpunkt 216h:
Phenylthiocarbamyltyrosin, Lysophosphatidylcholin mit Acylrest C28:1, wobei die Zahl nach "C" im Lysophosphatidylcholin die Anzahl der Kohlenstoffatome im Rest darstellt, und die Zahl nach dem Doppelpunkt die Anzahl der Doppelbindungen im Rest darstellt, Phenylalanin, Methioninsulfoxid und Putrescin und spezifisch für entzündungsbedingte Hirnschäden ist, und ein Molekulargewicht von weniger als 1500 Dalton aufweist, die Schritte umfassend:
a) Auswählen weiterer Verbindungen aus einer endogenen Ziel-Metaboliten-Gruppe, bestehend aus: Acetylcarnitin; Dodecandioylcarnitin; Propionylcarnitin; Propenoylcarnitin; Butyrylcarnitin/Isobutyrylcarnitin; Butenoylcarnitin; Isovalerylcarnitin / 2-Methylbutyrylcarnitin / Valerylcarnitin; Glutaconylcarnitin / Mesaconylcarnitin; Glutarylcarnitin; Tetradecenoylcarnitin; 3-Hydroxyhexadecanoylcarnitin; Triglylcarnitin/ 3-Methylcrotonylcarnitin; Phosphatidylcholin mit Diacylrest Summe C24:0; Phosphatidylcholin mit Diacylrest-Summe C28:1; Phosphatidylcholin mit Diacylrest-Summe C30:0; Phosphatidylcholin mit Diacylrest-Summe C32:2; Phosphatidylcholin mit Diacylrest-Summe C34:2; Phosphatidylcholin mit Diacylrest-Summe C34:3; Phosphatidylcholin mit Diacylrest-Summe C34:4; Phosphatidylcholin mit Diacylrest-Summe C36:0; Phosphatidylcholin mit Diacylrest-Summe C36:1; Phosphatidylcholin mit Diacylrest-Summe C36:2; Phosphatidylcholin mit Diacylrest-Summe C38:3; Phosphatidylcholin mit Diacylrest-Summe C38:6; Phosphatidylcholin mit Diacylrest-Summe C40:2; Phosphatidylcholin mit Diacylrest-Summe C40:5; Phosphatidylcholin mit Diacylrest-Summe C40:6; Phosphatidylcholin mit Acylalkylrest-Summe C30:1; Phosphatidylcholin mit Acylalkylrest-Summe C36:1; Phosphatidylcholin mit Acylalkylrest-Summe C36:2; Phosphatidylcholin mit Acylalkylrest-Summe C38:1; Phosphatidylcholin mit Acylalkylrest-Summe C38:2; Phosphatidylcholin mit Acylalkylrest-Summe C40:2; Tryptophan; Kynurenin; asymmetrisches Dimethylarginin; symmetrisches Dimethylarginin; Gesamtdimethylarginin; Phenylthiocarbamyl-methionin; Phenylthiocarbamylphenylalanin; Phenylthiocarbamylserin; Phenylthiocarbamylglycin; Glycin; Serin; Prolin; Valin; Tyrosin, Citrullin;
b) Messen mindestens einer der Parameter, ausgewählt aus der Gruppe bestehend aus: Konzentration, Niveau oder Menge jeder spezifischen Verbindung der Vielzahl von Verbindungen in der Probe, qualitatives und/oder quantitatives molekulares Muster und/oder molekulare Signatur; und Speichern des erhaltenen Wertesatzes in einer Datenbank;
c) Kalibrieren der Werte durch Vergleichen von Referenzparametern aus klinisch positiv bestätigten entzündungsbedingten Hirnschäden bei Frühgeborenen und/oder klinisch negativ bestätigten entzündungsbedingten Hirnschäden bei Frühgeborenen;
d) Vergleichen der Messwerte in der Probe mit den kalibrierten Werten, um zu beurteilen, ob der frühgeborene Patient wahrscheinlich eine entzündungsbedingte Hirnschädigung entwickeln wird oder ob es unwahrscheinlich ist, dass sich eine entzündungsbedingte Hirnschädigung entwickelt.

2. Verfahren nach Anspruch 1, wobei eine entzündungsbedingte Hirnschädigung eine infektionsassoziierte Hirnschädigung und/oder eine sepsisassoziierte Hirnschädigung umfasst.

3. Verfahren nach Anspruch 1 oder 2, wobei die quantitative Erfassung die Erstellung eines Metabolomikprofils umfasst, das durch ein quantitatives Metabolomikprofilanalyseverfahren erreicht wird, das die Erzeugung von Intensitätsdaten für die Quantifizierung der endogenen Metaboliten durch Massenspektrometrie (MS), insbesondere durch Massenspektrometrie mit hohem Durchsatz, vorzugsweise durch MS-Technologien wie Matrix Assisted Laser Desorption/Ionisation (MALDI), Elektrosprayionisierung ESI), Chemische Ionisierung unter Atmosphärendruck (APCI), ¹H-, ¹³C-, und/oder ³¹P-kernmagnetische Resonanzspektroskopie (NMR), gegebenenfalls MS-gekoppelt, Bestimmung der Metabolitenkonzentrationen unter Verwendung von MS-Technologien und/oder separationsgekoppelte Verfahren, insbesondere Flüssigchromatographie (LC-MS), Gaschromatographie (GC-MS) oder Kapillarelektrophorese (CE-MS).

4. Verfahren nach irgendeinem der Ansprüche 1 bis 3, wobei Intensitätsdaten des Metabolomikprofils mit einem Satz endogener Housekeeper-Metaboliten normalisiert werden, indem detektierte Intensitäten der ausgewählten endogenen Ziel-Metaboliten in Beziehung gesetzt werden, die für eine entzündungsbedingte Hirnschädigung zu Intensitäten der endogenen Housekeeper-Metaboliten prädiktiv sind.

5. Verfahren nach Anspruch 4, wobei die endogenen Housekeeper-Metaboliten aus der Gruppe ausgewählt sind, die aus solchen endogenen Metaboliten besteht, die Stabilität gemäß statistischen Stabilitätsmaßen aufweisen, die aus der Gruppe ausgewählt sind, die aus Variationskoeffizient (CV) von Rohintensitätsdaten, Standardabweichung (SD) von logarithmischen Intensitätsdaten, Stabilitätsmaß (M) des geNorm-Algorithmus oder Stabilitätsmaßwert (rho) des NormFinder-Algorithmus besteht.

6. Verfahren nach irgendeinem der Ansprüche 1 bis 5, wobei ein Panel von endogenen prädiktiven Referenz-Ziel-Metaboliten oder Derivaten davon etabliert wird durch:
a) mathematische Vorverarbeitung der Intensitätswerte, die zur Erzeugung der Metabolomikprofile erhalten wurden, um technische Fehler zu reduzieren, die den Messverfahren zur Erzeugung der Metabolomikprofile inhärent sind;
b) Auswahl mindestens eines geeigneten Klassifizierungsalgorithmus aus der Gruppe bestehend aus logistischer Regression, (diagonaler) linearer oder quadratischer Diskriminanzanalyse (LDA, QDA, DLDA, DQDA), Perzeptron, geschrumpfter Zentroiden, regularisierter Diskriminanzanalyse (RDA), Zufallswäldern (RF), neuronalen Netzen (NN), Bayesschen Netzen, versteckten Markov-Modellen, Unterstützungsvektormaschinen (SVM), generalisierte partielle Least Squares (GPLS), Partitionierung um Medoide (PAM), induktive Logikprogrammierung (ILP), generalisierte additive Modelle, Gaußsche Prozesse, regularisierte Least Square Regression, selbstorganisierende Karten (SOM), rekursive Partitionierungs- und Regressionsbäume, K-nearest neighbour Klassifikatoren (K-NN), Fuzzy Klassifikatoren, Bagging, Boosting und naiven Bayes; und Anwenden des ausgewählten Klassifikator-Algorithmus auf die vorverarbeiteten Daten von Schritt a);
c) die Klassifikatoralgorithmen aus Schritt b) auf mindestens einem Trainingsdatensatz trainiert werden, der vorverarbeitete Daten von Probanden enthält, die entsprechend ihrer Wahrscheinlichkeit, eine entzündungsbedingte Hirnschädigung zu entwickeln, in Klassen eingeteilt sind, um eine Klassifikatorfunktion auszuwählen, um die vorverarbeiteten Daten auf der Wahrscheinlichkeit abzubilden;
d) Anwenden der trainierten Klassifikator-Algorithmen aus Schritt c) auf einen vorverarbeiteten Datensatz eines Probanden mit unbekannter Wahrscheinlichkeit einer entzündungsbedingten Hirnschädigung und Verwenden der trainierten Klassifikator-Algorithmen zur Vorhersage der Klassen-Label des Datensatzes, um die Wahrscheinlichkeit vorherzusagen, dass ein Proband eine entzündungsbedingte Hirnschädigung entwickelt.

7. Verfahren nach irgendeinem der Ansprüche 1 bis 6, wobei die endogenen prädiktiven Ziel-Metaboliten zum leichteren und/oder empfindlicheren Nachweis mittels chemisch modifizierter Derivate davon, wie Phenylisothiocyanaten für Aminosäuren, nachgewiesen werden.

8. Verfahren nach irgendeinem der Ansprüche 1 bis 7, wobei die Vielzahl von endogenen prädiktiven Ziel-Metaboliten oder Derivaten davon 3 bis 55, insbesondere 3 bis 40, vorzugsweise 3 bis 30, bevorzugt 3 bis 25, bevorzugt 3 bis 25, besonders bevorzugt 3 bis 22, insbesondere bevorzugt 5 bis 40 endogene Ziel-Metaboliten umfasst.

9. Verfahren nach irgendeinem der Ansprüche 1 bis 8, wobei Laktat und/oder freies Carnitin als zusätzliche(r) Ziel-Metabolit(en) verwendet werden.

10. Verfahren nach irgendeinem der Ansprüche 1 bis 9, wobei ein Metabolomikprofil der Gruppe der Verbindungen der endogenen Metaboliten mit dem amplitudenintegrierten Elektroenzephalogramm (aEEG) und/oder mit dem Volumen der grauen Substanz und/oder dem Volumen der weißen Substanz korreliert wird.

11. Verwendung einer Vielzahl von mindestens den folgenden fünf endogenen Ziel-Metaboliten der Auswahl zum Zeitpunkt 216h: Phenylthiocarbamyltyrosin, Lysophosphatidylcholin mit Acylrest C28:1, wobei die Zahl nach "C" im Lysophosphatidylcholin die Anzahl der Kohlenstoffatome im Rest darstellt und die Zahl nach dem Doppelpunkt die Anzahl der Doppelbindungen im Rest darstellt, Phenylalanin, Methioninsulfoxid und Putrescin,
zur Vorhersage der Wahrscheinlichkeit eines Auftretens einer entzündungsassoziierten Hirnschädigung bei Frühgeborenen mit einer Genauigkeit von mehr als 70 % aus einer Blutprobe in vitro, wobei weitere Metaboliten ausgewählt sind aus der Gruppe, bestehend aus: Acetylcarnitin; Dodecandioylcarnitin; Propionylcarnitin; Propenoylcarnitin; Butyrylcarnitin/Isobutyrylcarnitin; Butenoylcarnitin; Isovalerylcarnitin / 2-Methylbutyrylcarnitin /Valerylcarnitin; Glutaconylcarnitin / Mesaconylcarnitin; Glutarylcarnitin; Tetradecenoylcarnitin; 3-Hydroxyhexadecanoylcarnitin; Triglylcarnitin/ 3-Methylcrotonylcarnitin; Phosphatidylcholin mit Diacylrest Summe C24:0; Phosphatidylcholin mit Diacylrest-Summe C28:1; Phosphatidylcholin mit Diacylrest-Summe C30:0; Phosphatidylcholin mit Diacylrest-Summe C32:2; Phosphatidylcholin mit Diacylrest-Summe C34:2; Phosphatidylcholin mit Diacylrest-Summe C34:3; Phosphatidylcholin mit Diacylrest-Summe C34:4; Phosphatidylcholin mit Diacylrest-Summe C36:0; Phosphatidylcholin mit Diacylrest Summe C36:1; Phosphatidylcholin mit Diacylrest Summe C36:2; Phosphatidylcholin mit Diacylrest Summe C38:3; Phosphatidylcholin mit Diacylrest Summe C38:6; Phosphatidylcholin mit Diacylrest Summe C40:2; Phosphatidylcholin mit Diacylrest-Summe C40:5; Phosphatidylcholin mit Diacylrest-Summe C40:6; Phosphatidylcholin mit Acylalkylrest-Summe C30:1; Phosphatidylcholin mit Acylalkylrest-Summe C36:1; Phosphatidylcholin mit Acylalkylrest Summe C36:2; Phosphatidylcholin mit Acylalkylrest Summe C38:1; Phosphatidylcholin mit Acylalkylrest Summe C38:2; Phosphatidylcholin mit Acylalkylrest Summe C40:2; Tryptophan; Kynurenin; asymmetrisches Dimethylarginin; symmetrisches Dimethylarginin; Gesamtdimethylarginin; Phenylthiocarbamylmethionin; Phenylthiocarbamylphenylalanin; Phenylthiocarbamylserin; Phenylthiocarbamyl-glycin; Glycin; Serin; Prolin; Valin; Tyrosin, Citrullin.

12. Verwendung nach Anspruch 11, wobei Laktat und/oder freies Carnitin als zusätzliche® Metabolit(e) verwendet werden.

13. Verwendung nach Anspruch 11 oder 12, wobei die Probe Blutplasma ist.

## Revendications

1. Procédé de prédiction de la probabilité d'une lésion cérébrale associée à une inflammation chez des nouveau-nés prématurés, avec une précision supérieure à 70 %,
**caractérisé par**
la détection quantitative *in vitro* dans au moins un échantillon de sang d'un patient d'une pluralité d'au moins les cinq composés suivants d'une sélection au point de temps de 216 h :
la phénylthiocarbamyl-tyrosine, la lysophosphatidylcholine avec un résidu d'acyle C28:1, dans lequel le nombre suivant «C» dans la lysophosphatidylcholine représente le nombre d'atomes de carbone dans le résidu, et le nombre après le deux-points représente le nombre de doubles liaisons dans le résidu, la phénylalanine, le sulfoxyde de méthionine et la putrescine, et spécifiques d'une lésion cérébrale associée à une inflammation, et ayant une masse moléculaire inférieure à 1500 Daltons comprenant les étapes suivantes :
a) la sélection d'autres composés parmi un groupe de métabolites cibles endogènes constitué de : l'acétylcarnitine ; la dodécanedioylcarnitine ; la propionylcarnitine ; la propénoylcarnitine ; la butyrylcarnitine/l'isobutyrylcarnitine ; la buténoylcarnitine ; l'isovalérylcarnitine/la 2-méthyl-butyrylcarnitine/la valérylcarnitine ; la glutaconylcarnitine/la mésaconylcarnitine ; la glutarylcarnitine ; la tétradécénoylcarnitine ; la 3-hydroxyhexadécanoylcarnitine ; la triglylcarnitine/la 3-méthyl-crotonylcarnitine ; la phosphatidylcholine avec la somme des résidus diacyle C24:0 ; la phosphatidylcholine avec la somme des résidus diacyle C28:1 ; la phosphatidylcholine avec la somme des résidus diacyle C30:0 ; la phosphatidylcholine avec la somme des résidus diacyle C32:2 ; la phosphatidylcholine avec la somme des résidus diacyle C34:2 ; la phosphatidylcholine avec la somme des résidus diacyle C34:3 ; la phosphatidylcholine avec la somme des résidus diacyle C34:4 ; la phosphatidylcholine avec la somme des résidus diacyle C36:0 ; la phosphatidylcholine avec la somme des résidus diacyle C36:1 ; la phosphatidylcholine avec la somme des résidus diacyle C36:2 ; la phosphatidylcholine avec la somme des résidus diacyle C38:3 ; phosphatidylcholine avec la somme des résidus diacyle C38:6 ; la phosphatidylcholine avec la somme des résidus diacyle C40:2 ; la phosphatidylcholine avec la somme des résidus diacyle C40:5 ; la phosphatidylcholine avec la somme des résidus diacyle C40:6 ; la phosphatidylcholine avec la somme des résidus acyl-alkyle C30:1 ; la phosphatidylcholine avec la somme des résidus acyl-alkyle C36:1 ; la phosphatidylcholine avec la somme des résidus acyl-alkyle C36:2 ; la phosphatidylcholine avec la somme des résidus acyl-alkyle C38:1 ; la phosphatidylcholine avec la somme des résidus acyl-alkyle C38:2 ; la phosphatidylcholine avec la somme des résidus acyl-alkyle C40:2 ; le tryptophane ; la kynurénine ; la diméthylarginine asymétrique ; la diméthylarginine symétrique ; la diméthylarginine totale ; la phénylthiocarbamyl-méthionine ; la phénylthiocarbamyl-phénylalanine ; la phénylthiocarbamyl-sérine ; la phénylthiocarbamyl-tyrosine ; la phénylthiocarbamyl-glycine ; la glycine ; la sérine ; la proline ; la valine ; la tyrosine, la citrulline ;
b) la mesure d'au moins l'un des paramètres sélectionnés dans le groupe constitué de : la concentration, le taux ou la quantité de chaque composé spécifique de ladite pluralité de composés dans ledit échantillon, le profil moléculaire qualitatif et/ou quantitatif et/ou la signature moléculaire ; et le stockage de l'ensemble de valeurs obtenu dans une base de données ;
c) l'étalonnage desdites valeurs par comparaison de paramètres de référence positifs de lésion cérébrale associée à une inflammation confirmée cliniquement chez des nouveau-nés prématurés et/ou négatifs de lésion cérébrale associée à une inflammation confirmée cliniquement chez des nouveau-nés prématurés ; et
d) la comparaison desdites valeurs mesurées dans l'échantillon aux valeurs étalonnées, afin d'estimer si le patient nouveau-né prématuré est susceptible de développer une lésion cérébrale associée à une inflammation ou n'est pas susceptible de développer une lésion cérébrale associée à une inflammation.

2. Procédé selon la revendication 1, dans lequel la lésion cérébrale associée à une inflammation comprend une lésion cérébrale associée à une infection et/ou une lésion cérébrale associée à une septicémie.

3. Procédé selon la revendication 1 ou 2, dans lequel ladite détection quantitative comprend l'établissement d'un profil métabolomique qui est obtenu par un procédé d'analyse quantitative du profil métabolomique comprenant la production de données d'intensité pour la quantification desdits métabolites endogènes par spectrométrie de masse (MS), en particulier, par spectrométrie de masse à haut débit, de préférence par des technologies de MS telles que la désorption et l'ionisation par laser assistées par matrice (MALDI), l'ionisation par électronébulisation (ESI), l'ionisation chimique à pression atmosphérique (APCI), la spectroscopie par résonance magnétique nucléaire (RMN) de ¹H, ¹³C et/ou ³¹P, éventuellement couplée à la MS, la détermination des concentrations des métabolites par l'utilisation de technologies de MS et/ou de procédés couplés à une séparation, en particulier la chromatographie liquide (LC-MS), la chromatographie gazeuse (GC-MS), ou l'électrophorèse capillaire (CE-MS).

4. Procédé selon l'une quelconque des revendications 1 à 3, dans lequel les données d'intensité dudit profil métabolomique sont normalisées avec un ensemble de métabolites domestiques endogènes par la mise en relation des intensités détectées des métabolites cibles endogènes sélectionnés prédisant une lésion cérébrale associée à une inflammation avec les intensités desdits métabolites domestiques endogènes.

5. Procédé selon la revendication 4, dans lequel lesdits métabolites domestiques endogènes sont sélectionnés dans le groupe constitué de ces métabolites endogènes qui présentent une stabilité conformément aux mesures statistiques de stabilité sélectionnées dans le groupe constitué du coefficient de variation (CV) des données brutes d'intensité, de l'écart-type (ET) des données logarithmiques d'intensité, de la mesure de stabilité (M) de l'algorithme geNorm ou de la valeur de la mesure de stabilité (rho) de l'algorithme NormFinder.

6. Procédé selon l'une quelconque des revendications 1 à 5, dans lequel un panel de métabolites cibles prédictifs endogènes de référence ou leurs dérivés est établi par :
a) le prétraitement mathématique des valeurs d'intensité obtenues pour produire les profils métabolomiques afin de réduire les erreurs techniques intrinsèques aux procédures de mesure utilisées pour produire les profils métabolomiques ;
b) la sélection d'au moins un algorithme de classification approprié dans le groupe constitué de la régression logistique, l'analyse discriminante linéaire ou quadratique (diagonale) (LDA, QDA, DLDA, DQDA), le perceptron, l'analyse discriminante régularisée par les centroïdes rétrécis (RDA), les forêts aléatoires (RF), les réseaux de neurones (NN), les réseaux bayésiens, les modèles de Markov cachés, les séparateurs à vaste marge (SVM), les moindres carrés partiels généralisés (GPLS), le partitionnement autour des médoïdes (PAM), la programmation logique inductive (ILP), les modèles additifs généralisés, les procédés gaussiens, la régression des moindres carrés régularisée, les cartes d'auto-organisation (SOM), le partitionnement récursif et les arbres de régression, les classifieurs des K plus proches voisins (K-NN), les classifieurs flous, de bagging, de boosting et naïfs de Bayes ; et l'application dudit algorithme classifieur sélectionné auxdites données prétraitées de l'étape a) ;
c) lesdits algorithmes classifieurs de l'étape b) étant entraînés sur au moins un ensemble de données d'entraînement contenant des données prétraitées provenant de sujets divisés en classes selon leur probabilité de développer une lésion cérébrale associée à une inflammation, afin de sélectionner une fonction de classifieur pour faire correspondre lesdites données prétraitées à ladite probabilité ;
d) l'application desdits algorithmes classifieurs entraînés de l'étape c) à un ensemble de données prétraitées d'un sujet avec une probabilité inconnue de lésion cérébrale associée à une inflammation, et l'utilisation des algorithmes classifieurs entraînés pour prédire l'étiquette de classe dudit ensemble de données afin de prédire la probabilité pour un sujet de développer une lésion cérébrale associée à une inflammation.

7. Procédé selon l'une quelconque des revendications 1 à 6, dans lequel lesdits métabolites cibles prédictifs endogènes pour une détection plus aisée et/ou plus sensible sont détectés au moyen de leurs dérivés modifiés chimiquement, tels que des isothiocyanates de phényle pour les acides aminés.

8. Procédé selon l'une quelconque des revendications 1 à 7, dans lequel ladite pluralité des métabolites cibles prédictifs endogènes ou de leurs dérivés comprend 3 à 55, en particulier 3 à 40, de préférence 3 à 30, de façon préférée 3 à 25, de façon davantage préférée 3 à 22, de façon particulièrement préférée 5 à 40 métabolites cibles endogènes.

9. Procédé selon l'une quelconque des revendications 1 à 8, dans lequel le lactate et/ou la carnitine libre est/sont utilisé(s) en tant que métabolite(s) cible(s) supplémentaire(s).

10. Procédé selon l'une quelconque des revendications 1 à 9, dans lequel un profil métabolomique dudit groupe de composés métabolites endogènes est mis en corrélation avec l'électroencéphalogramme à amplitude intégrée (aEEG) et/ou avec le volume de matière grise et/ou avec le volume de matière blanche.

11. Utilisation d'une pluralité d'au moins les cinq métabolites cibles endogènes suivant de la sélection au point de temps de 216 h : la phénylthiocarbamyltyrosine, la lysophosphatidylcholine avec un résidu d'acyle C28:1, dans lequel le nombre suivant « C » dans la lysophosphatidylcholine représente le nombre d'atomes de carbone dans le résidu, et le nombre après le deux-points représente le nombre de doubles liaisons dans le résidu, la phénylalanine, le sulfoxyde de méthionine et la putrescine,
pour prédire la probabilité de l'installation d'une lésion cérébrale associée à une inflammation chez des nouveau-nés prématurés avec une précision supérieure à 70 %, à partir d'un échantillon de sang *in vitro,* dans lequel d'autres métabolites sont sélectionnés dans le groupe constitué de : l'acétylcarnitine ; la dodécanedioylcarnitine ; la propionylcarnitine ; la propénoylcarnitine ; la butyrylcarnitine/l'isobutyrylcarnitine ; la buténoylcarnitine ; l'isovalérylcarnitine/la 2-méthylbutyrylcarnitine/la valérylcarnitine ; la glutaconylcarnitine/la mésaconylcarnitine ; la glutarylcarnitine ; la tétradécénoylcarnitine ; la 3-hydroxyhexadécanoylcarnitine ; la triglylcarnitine/la 3-méthyl-crotonylcarnitine ; la phosphatidylcholine avec la somme des résidus diacyle C24:0 ; la phosphatidylcholine avec la somme des résidus diacyle C28:1 ; la phosphatidylcholine avec la somme des résidus diacyle C30:0 ; la phosphatidylcholine avec la somme des résidus diacyle C32:2 ; la phosphatidylcholine avec la somme des résidus diacyle C34:2 ; la phosphatidylcholine avec la somme des résidus diacyle C34:3 ; la phosphatidylcholine avec la somme des résidus diacyle C34:4 ; la phosphatidylcholine avec la somme des résidus diacyle C36:0 ; la phosphatidylcholine avec la somme des résidus diacyle C36:1 ; la phosphatidylcholine avec la somme des résidus diacyle C36:2 ; la phosphatidylcholine avec la somme des résidus diacyle C38:3 ; phosphatidylcholine avec la somme des résidus diacyle C38:6 ; la phosphatidylcholine avec la somme des résidus diacyle C40:2 ; la phosphatidylcholine avec la somme des résidus diacyle C40:5 ; la phosphatidylcholine avec la somme des résidus diacyle C40:6 ; la phosphatidylcholine avec la somme des résidus acyl-alkyle C30:1 ; la phosphatidylcholine avec la somme des résidus acyl-alkyle C36:1 ; la phosphatidylcholine avec la somme des résidus acyl-alkyle C36:2 ; la phosphatidylcholine avec la somme des résidus acyl-alkyle C38:1 ; la phosphatidylcholine avec la somme des résidus acyl-alkyle C38:2 ; la phosphatidylcholine avec la somme des résidus acyl-alkyle C40:2 ; le tryptophane ; la kynurénine ; la diméthylarginine asymétrique ; la diméthylarginine symétrique ; la diméthylarginine totale ; la phénylthiocarbamyl-méthionine ; la phénylthiocarbamylphénylalanine ; la phénylthiocarbamyl-sérine ; la phénylthiocarbamyl-tyrosine ; la phénylthiocarbamylglycine ; la glycine ; la sérine ; la proline ; la valine ; la tyrosine, la citrulline.

12. Utilisation selon la revendication 11, dans laquelle le lactate et/ou la carnitine libre est/sont utilisé(s) en tant que métabolite(s) cible(s) supplémentaire(s).

13. Utilisation selon la revendication 11 ou 12, dans laquelle l'échantillon est du plasma sanguin.
